# EUROPEAN PATENT SPECIFICATION

(11) **EP 3 107 553 B1**
(45) Date of publication and mention of the grant of the patent: **01.12.2021**
(21) Application number: 15705603.7
(22) Date of filing: 18.02.2015
(51) Int. Cl.: A61K 35/24, A61P 1/00

(54) **METHODS AND COMPOSITIONS FOR INTESTINAL MICROENVIRONMENT TRANSFER**
METHODEN UND ZUSAMMENSTZUNGEN ZUR ÜBERTRAGUNG VON DER DARMMIKROUMGEBUNG
PROCEDES ET COMPOSITIONS POUR TRANSFERER LE MICROENVIRONMENT INTESTINAL

(30) Priority: 18.02.2014 EP 14155648; 01.08.2014 EP 14179541
(43) Date of publication of application: 28.12.2016
(73) Proprietor: Universitätsklinikum Jena, 07747 Jena (DE)
(72) Inventor: WÄTZIG, Georg, 24106 Kiel (DE); SEEGERT, Dirk, 24229 Dänischenhagen (DE)
(74) Representative: Eisenführ Speiser
(86) International application number: PCT/EP2015/053433
(87) International publication number: WO 2015/124637

(56) References cited:
- EP-A2- 0 027 429
- WO-A1-00/04911
- WO-A1-2008/075863
- WO-A1-2012/122478
- WO-A1-2013/186355
- WO-A2-2012/016287
- WESTERBEEK E A M ET AL: "The effect of enteral supplementation of specific neutral and acidic oligosaccharides on the faecal microbiota and intestinal microenvironment in preterm infants", EUROPEAN JOURNAL OF CLINICAL MICROBIOLOGY & INFECTIOUS DISEASES, SPRINGER, BERLIN, DE, vol. 32, no. 2, 9 September 2012 (2012-09-09), pages 269-276, XP035167411, ISSN: 1435-4373, DOI: 10.1007/S10096-012-1739-Y
- None

## Description

### FIELD OF THE INVENTION

The present invention relates to novel methods and compositions for positively influencing or normalising the human intestinal microbiota. The invented technique is termed for this text "intestinal microenvironment transfer (IMEnT)" and comprises
- the production of a liquid preparation of stool from a healthy animal, especially mammal, and particularly pig or human,
- depletion of the microbiota from this liquid stool preparation, by filtration with a filter having a maximum pore size of ≤ 450 nm, resulting in a filtrate containing a mixture of metabolites, nutrients and mediators produced by both the intestinal microbiota and the body cells in the intestinal system of the donor, termed herein the "intestinal microenvironment",
- transfer of this microbiota-depleted stool preparation (termed herein the primary IMEnT preparation) or derivatives thereof (termed herein secondary IMEnT preparations, which can be contained in compositions and/or formulations and/or supplements according to the present invention) to a recipient of the same or another species suffering from intestinal dysbiosis in order to revitalise and/or normalise the recipient's endogenous microbiota.

In addition, the present invention relates to pharmaceutical compositions and formulations (e.g., for nasogastric, nasointestinal, rectal or oral application) as well as dietary supplements which specifically release (e.g., selectively release) the IMEnT preparations into the small intestine and/or the large intestine.

### BACKGROUND

Many inflammatory diseases of the intestinal wall are caused or influenced by changes in the intestinal microbiota and/or an impaired interaction between the intestinal microbiota and the intestines. Such intestinal inflammations occur in humans, e.g., inflammatory bowel diseases (IBD), such as Crohn's disease or ulcerative colitis, but also in other mammals (e.g., chronic idiopathic colitis in dogs). These diseases are based on complex immunological processes which are not fully understood. However, changes in, and impaired interactions of, the intestinal microbiota can also be causative factors in a number of other diseases. Examples include atopic diseases, such as atopic eczema, allergic conditions or asthma (see e.g., Bisgaard et al. 2011, J. Allergy Clin. Immunol. 128:646; lebba et al. 2011, Dig. Dis. 29:531; Abrahamsson et al. 2012, J. Allergy Clin. Immunol. 129:434; Candela et al. 2012, BMC Microbiol. 12:95; Olszak et al. 2012, Science 336:489), as well as metabolic diseases with an inflammatory component, such as arteriosclerosis with resulting coronary heart diseases, adiposity or diabetes (Ott et al. 2006, Circulation 113:929; Koren et al. 2011, PNAS 108 Suppl 1:4592; for reviews see Caesar et al. 2010, J. Intern. Med. 268:320; and Vrise 2010, Diabetologia 53:606).

Therapeutic intervention by establishment or re-establishment of a normal gut microbiota or by supplementation of beneficial bacteria has been shown to be efficacious in diverse disease models and in the respective human diseases. For example, Olszak et al. (Science 2012, 336:489) recently demonstrated that the pathological accumulation of invariant natural killer T cells in diseased organs in germ-free murine models of IBD or asthma can be prevented by colonising neonate mice with normal microbiota. In different diseases, studies have demonstrated beneficial effects of certain pre-, pro- or synbiotics. For example, lactobacilli can reduce blood cholesterol levels in obesity, but the mechanism is still not completely clear (reviewed by Caesar et al. 2010 J. Intern. Med. 268:320). In inflammatory bowel diseases, some probiotics like VSL#3 (a mixture of *Bifidobacterium breve, Bifidobacterium longum, Bifidobacterium infantis, Lactobacillus acidophilus, Lactobacillus plantarum, Lactobacillus paracasei, Lactobacillus delbrueckii ssp. bulgaricus and Streptococcus thermophilus)* have been successfully used in a limited number of clinical studies. It appears that the supplementation of at least several strains of bacteria is usually necessary to provide significant therapeutic benefits.

The transfer of feces from healthy donors to recipients suffering from a dysfunctional or pathological composition of intestinal microbiota (dysbiosis) has long been known in both human and veterinary medicine under names such as fecal microbial transfer (FMT), fecal (microbiota / microbial / bacterial) transplant(ation), stool transplant(ation), fecal enema or fecal bacteriotherapy (Bakken et al. 2011 Clin. Gastroenterol. Hepatol. 9:1044; Gough et al. 2011 Clin. Infect. Dis. 53:994; Borody & Khoruts 2012 Nat. Rev. Gastroenterol. Hepatol. 9:88; Hamilton et al. 2012 Am. J. Gastroenterol. 107:761). As artificial and/or synthetic stool, composed of a defined panel of fecal bacteria, may also be effective [e.g., the large Danish case series of rectal bacteriotherapy recently published by Tvede et al. (Clin. Microbiol. Infect. 21:48, 2015)], the term "microbial ecosystem therapeutics" (MET) is also used by some authors (Allen-Vercoe et al. 2012 Can. J. Gastroenterol. 26:457; WO 2013/037068 A1; Petrof & Khoruts 2014 Gastroenterology 146:1573). In order to clearly differentiate also in terms of acronyms, the present invention of intestinal microenvironment transfer is abbreviated IMEnT and not IMET.

FMT has mainly, and very successfully, been applied in recurrent *Clostridium difficile (C. difficile)* infections (CDI) (Hamilton et al. 2012 Am. J. Gastroenterol. 107:761; van Nood et al. 2013 New Engl. J. Med. 368:407). In a wider sense, FMT has been proven to be highly effective in reconstituting and normalising the intestinal microbiota after antibiotic interventions. However, recent case reports and a growing understanding of the physiological and pathophysiological importance of the gut microbiota suggest that the targeted manipulation of the intestinal microbiota by FMT or other techniques additionally holds great promise for the treatment of other chronic gastrointestinal infections (e.g., by *Yersinia* spp., *Campylobacter* spp., *Aeromonas* spp., *Escherichia coli, Cryptosporidium* spp., amoebae, Giardia, or chronic viral infections), small bowel bacterial overgrowth, IBD (such as Crohn's disease or ulcerative colitis), lymphocytic colitis, mucous colitis, collageneous colitis, microscopic colitis, antibiotic-associated colitis, diverticular disease, AIDS enteropathy, colon cancer, irritable bowel syndrome, spastic colon and idiopathic or simple constipation, but also for other diseases affecting organs beyond the gastrointestinal tract, such as sacroiliitis, the metabolic syndrome, obesity, diabetes, insulin resistance, non-alcoholic fatty liver disease, non-alcoholic steatohepatitis, cardiovascular diseases like arteriosclerosis and atherosclerosis, chronic fatigue syndrome, autoimmune diseases, idiopathic thrombocytopenic purpura, allergic diseases and neurodevelopmental or neurodegenerative disorders such as Parkinson's disease, multiple sclerosis or autism (Borody & Khoruts 2012 Nat. Rev. Gastroenterol. Hepatol. 9:88; Hamilton et al. 2012 Am. J. Gastroenterol. 107:761; Cotillard et al. 2013 Nature 500:585; Le Chatelier et al. 2013 Nature 500:541; Shanahan 2013 Curr. Opin. Gastroenterol. 29:49; Ridaura et al. 2013 Science 341:1079; Smits et al. 2013 Gastroenterology 145:946; US 5443826; WO 2011/094027A1; WO 2012/122478A1). Recurrent CDI can also be a consequence of the dysbiosis in IBD, which is illustrated by the fact that approximately 30% of the CDI patients of a recently published systematic FMT study also had underlying IBD (Hamilton et al. 2012 Am. J. Gastroenterol. 107:761).

Both in the published case reports and small open-label studies as well as in a recent publication of a larger randomised study, FMT has been highly effective against recurrent CDI with disease resolution rates of about 90% (Gough et al. 2011 Clin. Infect. Dis. 53:994; Borody & Khoruts 2012 Nat. Rev. Gastroenterol. Hepatol. 9:88; Hamilton et al. 2012 Am. J. Gastroenterol. 107:761; Kassam et al. 2013 Am. J. Gastroenterol. 108:500; van Nood et al. 2013 New Engl. J. Med. 368:407; Youngster et al. 2014 JAMA 312:1772).

The current state of the art of FMT is based on two main premises, namely
1) that patients with dysbiosis have either completely lost their healthy microbiota or that their microbiota is unable to regain its normal functionality, and
2) that these pathogenic changes and/or a loss of diversity in the intestinal microbiota can be corrected by transferring a stable, viable, diverse and healthy microbial community contained in stool preparations from a healthy donor.

Current FMT techniques therefore all aim at maintaining as much of the microbial diversity and natural composition of the donor microbiota as possible.

The conventional routes of FMT are either rectal (*e*.*g*., via colonoscopy or retention enema) or nasal (e.g., via nasogastric, nasoduodenal, nasojejunal or nasoileal tube infusion). While the recent meta-analysis of Kassam *et al.* suggests that the rectal route might be safer and more efficacious (Kassam et al. 2013 Am. J. Gastroenterol. 108:500), other authors have not seen superiority of any method (Bakken et al. 2011 Clin. Gastroenterol. Hepatol. 9:1044; Borody & Khoruts 2012 Nat. Rev. Gastroenterol. Hepatol. 9:88). In any case, the condition of the patient requires individual risk assessments and decisions about the best route of administration (Kassam et al. 2013 Am. J. Gastroenterol. 108:500). Recently, the successful oral application of capsulised, frozen fecal microbiota has been reported with a 90% success rate corresponding to the published average of conventional FMT (Youngster et al. 2014 JAMA 312:1772).

Several commercial approaches and patent applications aim at using variants of FMT. Examples of patents or patent applications are US 5443826, WO 2011/094027A1 and WO 2012/122478A1.

The RBX2660 enema with fecal bacteria (Rebiotix, Roseville, MN) is being tested in phase II for CDI, and Monarch Labs (Irvine, CA) have announced in 2013 to develop both a cGMP processing and banking service for autologous FMT transplantation and an FMT product for allograft transplantation, which shall be cGMP-processed and screened for major pathogens (Medical Microbiota^{™}). The non-profit organisation OpenBiome (Microbiome Health Research Institute, Inc., Medford. MA, USA) delivers frozen FMT preparations and also works on capsule formats (www.openbiome.org).

However, despite the superb efficacy and the good short-term safety profile of FMT, the current state of the art bears some major problems and unknown long-term risks (Kassam et al. 2013 Am. J. Gastroenterol. 108:500; Smits et al. 2013 Gastroenterology 145:946; Petrof & Khoruts 2014 Gastroenterology 146:1573). Recruiting highly characterised and standardised populations of stool donors akin to blood donors and using microbe-conserving freezing procedures for the stool preparations can largely overcome the problem of emergency availability and some of the safety issues (Bakken et al. 2011 Clin. Gastroenterol. Hepatol. 9:1044; Hamilton et al. 2012 Am. J. Gastroenterol. 107:761). However, even the most rigorous and costly donor screening procedures (as defined in the current guidelines by Bakken et al. 2011 Clin. Gastroenterol. Hepatol. 9:1044 or in WO 2012/122478A1) cannot exclude the risk of transferring unknown or undetectable defects of the transplanted "microbial organ" to the recipient, such as unknown pathogenic bacteria or viruses, antibiotic-resistance determinants, as well as bacterial or viral risk factors for metabolic diseases, cancer, atopy or autoimmunity (Hamilton et al. 2012 Am. J. Gastroenterol. 107:761; Kassam et al. 2013 Am. J. Gastroenterol. 108:500; Ridaura et al. 2013 Science 341:1079; Smits et al. 2013 Gastroenterology 145:946; Petrof & Khoruts 2014 Gastroenterology 146:1573). For example, recent studies have demonstrated disease-specific intestinal viromes in IBD (Norman et al. 2015 Cell 160:447) and that some commensal bacteria can initiate and drive colonic carcinogenesis through a bystander effect (Wang et. al. 2015 Gut 64:459). Moreover, a recent case of probably FMT-induced obesity after transfer of microbiota from an obesity-prone donor gained significant interest in the media (Alang & Kelly 2015 Open Forum Infect. Dis. 2:10.1093/ofid/ofv004). This case extended the findings of Ridaura et al. (Science 341:1079, 2013), who demonstrated transmissible metabolic effects of the microbiota of human twins discordant for obesity. In addition, transfer of the microbiota to an immunodeficient or immunosuppressed recipient is often too dangerous, which excludes some patients in critical condition (Bakken et al. 2011 Clin. Gastroenterol. Hepatol. 9:1044). In addition, the techniques of FMT are also not free of risks, e.g., intestinal perforations during colonoscopy or complications with nasogastric or nasointestinal tubes, and not very comfortable for the recipient (Kassam et al. 2013 Am. J. Gastroenterol. 108:500). Finally, the aesthetic aspects of the procedure, the so-called 'yuck-factor' for both the patients and the medical staff, could prevent FMT from becoming a standard therapeutic option for patients in a less desperate condition than severe CDI (Borody & Khoruts 2012 Nat. Rev. Gastroenterol. Hepatol. 9:88; WO 2012/122478A1, p. 3 line 32 to p. 4 line 6).

Accordingly, the technical problem underlying the present invention is to provide novel techniques and formulations to use beneficial aspects of FMT without its inherent drawbacks and risks.

The solution to said technical problem is achieved by providing the embodiments characterized in the claims.

### SUMMARY OF THE INVENTION

The object of the present invention is to provide new forms of treatments for the therapy and/or prophylaxis of diseases in humans and animals associated with changes in the intestinal microbiota and/or an impaired interaction between the intestinal microbiota and the intestines.

According to the invention, the above problem is solved in general by a preparation (e.g., a filtrate and/or a composition and/or a formulation and/or a supplement, especially a pharmaceutical composition or formulation or dietary supplement), containing an IMEnT preparation as described herein, which can positively influence and/or revitalise and/or normalise the intestinal microbiota. In preferred embodiments, the IMEnT preparation is administered to locally influence the intestinal mucosa and the intestinal microbiota. For example, the IMEnT preparation is formulated to be administered selectively in the small intestine and/or large intestine, preferably in the lower small intestine or colon, where the intestinal microbiota to be modified is located. The IMEnT preparation can be derived directly from fresh, cooled or cryopreserved stool or stool pools, from cultured and/or fermented stools or stool pools as well as from artificial and/or synthetic stool preparations consisting of a defined panel of fecal microbiota. Combinations with one or more other therapeutic substances and preparations, such as - but not limited to - probiotics, prebiotics, synbiotics, proteins and/or enzymes supporting probiotics, antibiotic, antimycotic, antiprotozoal, antihelminthic, antiviral or anti-inflammatory agents; aminosalicylates; nicotinic acid; nicotinamide; tryptophan; compounds that convert in the body of an animal or human into nicotinic acid, nicotinamide or tryptophan; nicotinamide adenine dinucleotide (NAD); nicotinamide adenine dinucleotide phosphate (NADP); intermediates in the biosynthesis of NAD or NADP; tryptophan dipeptides; short-chain fatty acids; medium-chain fatty acids; systemic or topical corticosteroids; β₂-adrenergic receptor agonists; theophylline and other substances of the xanthine family; statins; small molecules; peptides and biologicals, are also part of the present invention.

Accordingly, preparations (e.g., filtrates and/or compositions and/or formulations and/or supplements, especially pharmaceutical compositions and formulations or dietary supplements) are provided which contain IMEnT preparations. These act in a beneficial and/or anti-inflammatory manner on the microbiota in the small intestine and/or large intestine. It is preferred that such preparations and/or compositions and/or formulations and/or supplements are, *e.g*., ready-to-use liquids, concentrates (for dilution), lyophilisates (for reconstitution) or other formulations for use in nasal (nasogastric or nasointestinal) or rectal applications. In another preferred embodiment, such preparations and/or compositions and/or formulations and/or supplements are suitable for oral administration with controlled and/or delayed release of the active ingredient(s) for specific local or topical efficacy in the small intestine and/or large intestine, preferably in the lower small intestine and/or colon. Exemplary conditions in which IMEnT preparations and/or compositions and/or formulations and/or supplements are applied include dysbiosis of the small and/or large intestine; antibiotic therapy (before, concomitantly or afterwards); therapy, prophylaxis or remission of inflammatory diseases of the small intestine; therapy, prophylaxis or remission of inflammatory diseases of the large intestine; prophylaxis of colon carcinoma; therapy or prophylaxis of obesity (by IMEnT from a slim donor); and therapy or prophylaxis of other diseases which result from changes in the intestinal microbiota and/or an impaired interaction between intestinal microbiota and intestines. The preparations and/or compositions and/or formulations and/or supplements of the present invention are also suitable for the (neo)rectal administration in the colon or pouch for the local and/or topical therapy of said conditions.

The invention also provides methods of preventing or treating one or more of the diseases and conditions described herein with a preparation and/or composition and/or formulation and/or supplement, e.g., a pharmaceutical composition or dietary supplement, described herein. In addition, the invention provides the use of a preparation and/or composition and/or formulation and/or supplement, e.g., a pharmaceutical composition or a composition suitable for dietary supplements, described herein in the manufacture of a medicament for preventing or ameliorating or treating one or more of the diseases, disorders and conditions described herein, and/or in the manufacture of a dietary supplement for helping to prevent or ameliorate one or more of the diseases, disorders and conditions described herein. Furthermore, the invention provides the use of a preparation and/or composition and/or formulation and/or supplement described herein for non-therapeutically positively influencing the intestinal microbiota of an animal, preferably of a mammal, in particular of a human.

Finally, the invention provides methods for producing a preparation (e.g., preparing a filtrate) from an animal, preferably a mammal, in particular a pig or human, stool sample and/or stool culture and/or artificial and/or synthetic stool.

### BRIEF DESCRIPTION OF THE DRAWINGS

Figure 1 shows the most abundant operational taxonomic units (OTUs) across all samples obtained from Patient 2 (see Example 5) and the stool donor immediately before IMEnT (day 0) and from the patient on days 7 and 43 after IMEnT. The microbiota of the patient was changed substantially as evidenced at day 7 after IMEnT, and the changes compared to the microbiota in its diseased status (day 0) remained well detectable also at day 43 after IMEnT.
Figure 2 shows that the stool microbiota of specific pathogen-free Göttingen minipigs bred and kept under good manufacturing practice (GMP) conditions has a low inter-individual variability and sufficient diversity on the phylum level.
Figure 3 shows that the stool microbiota of specific pathogen-free Göttingen minipigs bred and kept under good manufacturing practice (GMP) conditions has a low inter-individual variability and sufficient diversity also on the genus level.

### DETAILED DESCRIPTION

Herein, the words "preferred" or "preferably" refer to embodiments that may have certain benefits under certain circumstances, but other embodiments may also be preferred under the same or other circumstances. The recitation of one or more preferred embodiments does not imply exclusion of other useful embodiments from the scope of the invention. Terms like "comprises" and variations thereof do not have a limiting meaning in the description and claims. Citation of certain sections of documents from the literature does not imply that the rest of such documents is not relevant or not incorporated by reference. The recitations of numerical ranges by one or two endpoints includes all numbers subsumed within that range *(e.g.,* "1 to 10" includes 1, 2.4, 4.576, etc., and "lower than 1" includes all numbers smaller than 1). For any method disclosed or cited herein that includes discrete steps, the steps may be conducted in any feasible order, and any combination of two or more steps may be conducted simultaneously. Any example or list of examples should not be interpreted as a restriction of any kind or as an exclusive list.

The present invention pertains to a preparation from animal and/or artificial and/or synthetic and/or cultured and/or fermented stool, made by suspending the stool in a liquid and by depletion of the microbiota comprised in the suspension by filtrating the suspension with a filter having a maximum pore size of ≤ 450 nm for use as a medicine. Further features of the subject matter of the invention are defined in the claims. This preparation *per se* (independently of its purpose) is termed herein a primary IMEnT preparation.

In addition to that, the present invention pertains to a preparation from animal and/or artificial and/or synthetic and/or cultured and/or fermented stool, made by suspending the stool in a liquid and by depletion of the microbiota comprised in the suspension by filtrating the suspension with a filter having a maximum pore size of ≤ 450 nm and wherein the water content is ≤ 95 weight-%, preferably ≤ 80 weight-%, more preferably ≤ 65 weight-% and most preferably ≤ 50 weight-% relating to the total weight of the filtrate, and/or the microbiota and the virus load comprised in the suspension is depleted analogue to or by performing a second filtration with a maximum pore size of ≤ 20 nm. Such a preparation *per se* (independently of its purpose) is termed herein a secondary IMEnT preparation. A secondary IMEnT preparation according to the invention can be used as a medicine and/or as a dietary supplement. It goes without saying that primary IMEnT preparations as described herein can be used as dietary supplement, too.

A "depletion from the microbiota or the virus load analogue to a filtration with a certain pore size" means - as used in this text - that a depletion of living microorganisms and viruses respectively was effected as would have been by a filtration with the certain pore size. Such a depletion can be achieved, e.g., by irradiation, adsorption or relating techniques for reducing the amount of living microorganisms or viruses.

The invention pertains to a preparation in form of a filtrate.

For the preparations according to the invention, the animal is preferably a mammal and more preferably a pig or human.

In addition, the present invention pertains to a composition consisting of or comprising the preparation as defined herein before and/or herein after as active ingredient, wherein the composition is a pharmaceutical composition or a dietary supplement.

In this text, the term "pharmaceutical composition for treatment or prevention regimen", and in particular the term "pharmaceutical composition", has a broad meaning of a pharmaceutically and/or physiologically acceptable composition comprising the preparation according to the invention as active ingredient if a pharmaceutically effective amount is comprised, which includes but is not limited to pharmaceutical formulations in the sense of medicaments (drugs) and which also includes nutraceuticals, dietary supplements, and in its broadest sense may even include food ingredients and foods. Therefore, depending on the dose of the active substance(s) and the formulation, a pharmaceutical composition according to the present invention includes, but is not limited to, formulations as medicaments, nutraceuticals, dietary supplements, food ingredients and/or foods. Preferred are medicaments, nutraceuticals, and dietary supplements. Particularly preferred are pharmaceutical compositions as medicaments or dietary supplements.

Further, the present invention pertains to a composition consisting of or comprising the preparation as defined herein before and/or herein after as active ingredient, for positively influencing the intestinal microbiota of an animal, preferably a mammal, in particular a human.

Moreover the present invention pertains to a composition consisting of or comprising the preparation as defined herein before and/or herein after as active ingredient, for use in the therapy or prophylaxis of diseases that result from changes in the intestinal microbiota and/or an impaired interaction between intestinal microbiota and intestines.

Preferably, in the context of the invention, e.g., with regard to the filtrates, compositions, formulations, preparations, supplements, and/or uses thereof, and/or methods, the animal is a mammal, and in particular a pig or human. Preferred are embodiments of the invention, e.g., preparations and/or compositions and/or formulations and/or supplements and/or filtrates, wherein the stool is from a pooled sample or wherein preparations from individual stool samples are pooled to produce larger batches.

As used herein, the terms "artificial stool" and "synthetic stool" denote combinations and/or preparations and/or cultures of gut microbiota (e.g., cultured individually or together, e.g., in fermenters or artificial guts) in a composition comprising or consisting of preferably at least 5, more preferably at least 10, even more preferably at least 15 microbial species derivable from the stool of at least one healthy animal, preferably a mammal, and in particular a pig or human. Preferably, artificial stool and/or synthetic stool is composed and/or produced as described in WO 2013/037068 A1. More preferably, artificial and/or synthetic stool is a composition as described in one of or all of the definitions in the section starting on p. 2, line 15, and ending on p. 5, line 2 of this document. Machineries to produce artificial and/or synthetic stool are described in the literature [e.g., the large intestinal system (Minnekus et al. 1999 Appl. Microbiol. Biotechnol. 53:108) or the Robogut of the RePOOPulate system (Petrof et al. 2013 Microbiome 1:3; Petrof & Khoruts 2014 Gastroenterology 146:1573). All the techniques developed for human stool can easily be performed also with stool from other animals, e.g., with pig stool.

As used herein, the term "cultured stool" denotes the product of a cultivation process, which can be performed with natural and/or artificial and/or synthetic stool as described herein and in the literature.

As used herein, the term "fermented stool" denotes the product of a fermentation process, which can be performed with natural and/or artificial and/or synthetic stool as described herein and in the literature.

The present invention shows that the microbiota-depleted liquid phase of a stool preparation (i.e., a stool preparation from which the microbiota has been depleted and which contains a mixture of metabolites, nutrients and mediators produced by both the intestinal microbiota and the donor's body cells) from stool of an animal, especially mammal or human, donor can be sufficient to positively influence and/or revitalise and/or normalise the residual intestinal microbiota in a recipient, usually but not necessarily of the same species, suffering from intestinal dysbiosis. This novel IMEnT technique surprisingly disproves the current understanding that intestinal dysbiosis, e.g. in patients with CDI, can only be cured by transplanting the complex microbiota of a healthy donor.

As used herein, the term "dysbiosis" denotes a condition or disease in a subject, e.g., an animal, a mammal or human, suffering from a dysfunctional or pathological composition of intestinal microbiota.

The advantages of IMEnT over conventional FMT (fecal microbial transfer) are:
- no or low risk of transplanting unknown or undetected microbial pathogens or risk factors for diseases;
- in a preferred embodiment incorporating a second filtration step with a filter having a maximum pore size of ≤ 20 nm, a very low risk of transplanting intestinal viruses (microbiota-associated viruses are already largely eliminated together with the microbiota in previous centrifugation and/or filtration and/or other sterilisation steps such as gamma irradiation);
- in the case of nasal (nasogastric or nasointestinal) or rectal administration, the use of standardised and quality-controlled IMEnT preparations [e.g., ready-to-use liquids, concentrates (for dilution), lyophilisates (for reconstitution) or other formulations] enables immediate treatment of patients at any time without the need for individual donor screening and also allows a rapid and very streamlined risk assessment;
- in the case of oral administration of IMEnT formulations, the procedural risks and discomfort of conventional FMT procedures, such as bowel lavages, nasogastric or nasointestinal tubes, or colonoscopy, are not necessary;
- nasal or rectal administration of IMEnT formulations is better tolerated than FMT, as no FMT-typical side effects like elevated body temperature and/or short-term diarrhea were observed so far (see Example 5);
- repeated oral administration of IMEnT formulations is well tolerated and not limited with regard to frequence and/or duration;
- in the case of human or pig donors and human recipients, less disgust and better compliance with the procedure, as no microorganisms are transferred and, in the case of oral administration, no tubing is required as opposed to conventional FMT;
- previous, concomitant or subsequent antibiotic therapy does not affect therapeutic efficacy of IMEnT formulations, as they do not contain live microbiota;
- an easier production process, as the viability of the microbiota does not play a role for the final product (e.g., no purging of blenders or other production tools with inert gasses to maintain anaerobic conditions);
- less complexity and easier characterisation of the IMEnT formulations compared to FMT transplants.

Using pig stool instead of human stool for IMEnT preparations enables easier production of larger, safer, more homogeneous and better characterisable batches with perfect production conditions with regard to scalability, food regimen (controlled food and controlled supplementation of food ingredients, prebiotics, probiotics and the like) and without human risk factors such as known or unknown pathogens, in particular when specific pathogen-free (SPF) minipigs are used under GMP conditions. Pigs are very similar to humans in the anatomy, physiology and metabolism of their digestive system and are the best model species for emulating the human gastroenterological tract (Pang et al. 2007 ISME J. 1:156; Guilloteau et al. 2010 Nutr. Res. Rev. 23:4; Pedersen et al. 2013 BMC Microbiol. 13:30). As in humans (Eckburg et al. 2005 Science 308:1635), the two main phyla of bacteria in the gut microbiota of pigs are *Bacteroidetes* and *Firmicutes* (Leser et al. 2002 Appl. Environ. Microbiol. 68:673; Lamendella et al. 2011 BMC Microbiol. 11:103). The high similarity of the pig and human microbiota has been confirmed in the studies of the present invention, which supports the use of pig stool for IMEnT (see Example 7). Accordingly, a preferred preparation according to the invention is a preparation from pig stool.

In all subjects with dysbiosis, IMEnT, e.g., the preparations (e.g., filtrates) and/or compositions and/or formulations and/or supplements of the invention, is therefore suggested as a first-line treatment due to its superior safety profile. If IMEnT, e.g., the preparations (e.g., filtrates) and/or compositions and/or formulations and/or supplements of the invention should fail because the microbiota of the recipient is too damaged to recover in response to IMEnT, FMT can still be performed as a second-line therapy.

The complex mixture of substances contained in IMEnT preparations and/or formulations, e.g., the filtrates and/or compositions and/or supplements of the invention, mirrors the natural environment of the healthy gut microbiota. Primary IMEnT preparations and final preparations and/or compositions and/or formulations and/or supplements derived thereof may contain typical components of fecal water, such as - but not limited to - bile acids, heme, polyphenols, diverse fatty acids (especially short-chain and branched-chain fatty acids), amino acids, glycerol, glucose, ammonia, lactate, methylamine and trimethylamine (Jacobs et al. 2008 NMR Biomed. 21:615, see in particular Table 1 of this reference; Monleon et al. 2009 NMR Biomed. 22:342; Pearson et al. 2009 Nutr. Rev. 67:509) as well as debris from microbiota and host cells. In addition, such IMEnT preparations and/or compositions and/or formulations and/or supplements may also contain a host of other metabolic products from both the host and its microbiota as well as mediators determining the composition of the microbiota, such as antimicrobial or other peptides, proteins or other microbiota-regulating substances produced by the host and/or the host's microbiota. Moreover, IMEnT preparations may also contain oligonucleotides and/or polynucleotides derived from the host and/or the host's microbiota, which are either produced and/or released for a specific purpose or which result from cell and/or virus disruption and/or debris. Enrichment and/or purification of one or more substances, substance groups and/or fractions contained in IMEnT preparations for all uses according to the present invention is also within the scope of the invention. Preferred examples for such substance groups and/or fractions include, but are not limited to, antimicrobial peptides, oligonucleotides (e.g., those smaller than 30 base pairs or bases), bile acids, bile acid derivatives (e.g., secondary bile acids, see Buffie et al. 2015 Nature 517:205) or fatty acids (e.g., short chain fatty acids). Moreover, IMEnT preparations may also be enriched in such substances, substance groups and/or fractions, e.g., by adding such substances, substance groups and/or fractions either derived from IMEnT preparations or from synthesis or other sources.

For some of the components of IMEnT preparations and/or compositions and/or formulations and/or supplements, beneficial effects on intestinal homeostasis have been suggested, but not clearly proven. This is illustrated by the example of short-chain fatty acids (SCFA). It has been shown previously that fermentation of dietary polyphenols by a culture model of human intestinal microbiota stimulated proliferation of Bifidobacteria and decreased the ratio of Firmicutes to Bacteroidetes, and that the polyphenol metabolites (not the polyphenols themselves) induced short fatty acid production by the microbiota (Parkar et al. 2013 Anaerobe 23:12). IBD patients have significantly changed SCFA levels compared to healthy subjects, but despite numerous studies suggesting anti-inflammatory effects of SCFA in cell models, animal models and in patients, SCFA administration is not clearly beneficial in ulcerative colitis (Huda-Faujan et al. 2010 Open Biochem. J. 4:53; Vinolo et al. 2011 Nutrients 3:858; Soldavini & Kaunitz 2013 Dig. Dis. Sci. 58:2756). In contrast, the beneficial effect of IMEnT goes far beyond the action of individual substances or substance classes administered separately, because supplementation of such small parts of the whole intestinal microenvironment cannot exert the same balancing beneficial effect as the whole complex mixture or larger fractions or substance groups thereof. Similarly, probiotics containing a few bacterial strains can usually not substitute for the whole ecosystem of the gut microbiota transferred in FMT.

For clarification, the inventors use the following definitions:
Probiotic: ingestible live microbial cultures, which survive transit through the gastrointestinal tract and beneficially affect the host by improving its intestinal microbial balance. An example for a probiotic is VSL#3 (see above).

Prebiotic: non-digestible and selectively fermented food ingredient or supplement that allows specific changes in the composition and/or activity of the gastrointestinal microbiota which are beneficial for host well-being and health. Examples for prebiotics are resistant starch, fructo-oligosaccharides, galacto-oligosaccharides, xylo-oligosaccharides, polydextrose, lactulose, inulin or soluble fiber (e.g., psyllium husk or acacia fibers).Synbiotics: a combination of pro- and prebiotics.

As used herein, "positively or beneficially influencing the intestinal microbiota" refers to causing a change in the intestinal microbiota that has a positive impact on health, especially on one or more of the diseases and conditions described herein. For example, positive impacts are associated with reducing the number of pathogenic bacteria, reducing the ratio of pathogenic bacteria to beneficial bacteria, increasing the diversity of the microbiota, reducing the amount of inflammation that pathogenic microbiota may induce in the intestines, and partly or completely reverting pathological changes in the enterotype of the microbiota (e.g., enterotypes associated with *Bacteroides*, *Prevotella* and *Ruminococcus*)*.* In addition to *Clostridium difficile* and other pathogenic bacterial species mentioned herein, bacteria generally regarded as pathogenic in inflammatory bowel diseases include, for example, *Enterobacteriaceae* (*e.g., Escherichia coli*) with invasive properties or virulence factors, sulphide-producing *Desulfovibrio* spp. and *Fusobacterium* spp with invasive properties. Bacteria generally regarded as beneficial include species from the genera *Lactobacillus, Bifidobacterium* and *Faecalibacterium,* such as *L. casei, L. plantarum* and *F. prausnitzii.* For a recent overview of the gut microbiota in inflammatory bowel diseases, see Manichanh et al. (Nat. Rev. Gastroenterol. Hepatol. 2012, 9:599).

In short, IMEnT preferably comprises several highly standardised procedures for preparing stool samples, depletion of the intestinal microbiota from the liquid of the stool preparation i.e. by filtration, further processing of this liquid (the primary IMEnT preparation) into diverse secondary IMEnT preparations (e.g., by depleting removable intestinal viruses in a second filtration step, concentration, drying, etc.) and/or compositions and/or formulations and/or supplements, and finally administration of these preparations and/or compositions and/or formulations and/or supplements to subjects with dysbiosis (humans or other mammals). Preparation of stool samples for IMEnT comprises, but is not limited to, each one or a combination of the following procedural examples:
- preparing stool samples from a well-characterised donor population and processing these directly to IMEnT preparations, whereby samples and/or IMEnT preparations can be pooled at any stage of the process;
- preparing stool samples from a well-characterised donor population and optimising and/or expanding the stool samples or pools thereof by fermentation and/or addition of probiotics and/or prebiotics and/or synbiotics and/or an optimised standard stool and/or synthetic and/or artificial and/or fermented stool before processing them to IMEnT preparations;
- preparing artificial and/or synthetic stool preparations and cultures consisting of a defined panel of fecal microbiota as a source and/or combination element for IMEnT preparations;
- donating stool for oneself, *e.g*., before surgery and/or antibiotic treatments, for retransplantation of the intestinal microenvironment as IMEnT preparation and/or composition and/or formulation and/or supplement with or without additional steps of optimising one's IMEnT preparation by fermentation of one's stool and/or addition of probiotics and/or prebiotics and/or synbiotics and/or an optimised standard stool from a well-characterised donor population and/or synthetic and/or artificial and/or fermented stool as described herein;
- donating stool for oneself as described herein, for retransplantation of the own stool together with IMEnT preparations from, *e.g*., a well-characterised donor population and/or stool cultures and/or artificial and/or synthetic and/or fermented stool as described herein, with or without additional steps of optimising one's stool by fermentation of one's stool and/or addition of probiotics and/or prebiotics and/or synbiotics and/or an optimised standard stool from a well-characterised donor population and/or synthetic and/or artificial and/or fermented stool as described herein;
- removing detrimental ingredients from one's stool sample, such as pathogenic microbiota (*e.g*., *Clostridium difficile*) and/or toxins (*e.g*., *Clostridium difficile* toxins), and retransplantation of one's improved stool sample together with IMEnT preparations for optimal expansion of the improved stool;
- removing detrimental ingredients from one's stool sample, such as pathogenic microbiota (e.g., *Clostridium difficile*) and/or toxins (e.g., *Clostridium difficile* toxins), and additional steps of optimising one's IMEnT preparation by fermentation of one's purified stool sample and/or addition of probiotics and/or prebiotics and/or synbiotics and/or an optimised standard stool from a well-characterised donor population and/or synthetic and/or artificial and/or fermented stool as described herein.

In this regard, *e.g.*, in particular in regard of preparing filtrates, the present invention especially pertains to a method for preparing a preparation as defined herein before and/or herein after as active ingredient, the method comprising the steps of:
a) providing an animal, preferably a mammal, in particular a pig or human, stool sample,
b) suspending the stool sample,
c) depleting the microbiota comprised in the suspension by filtrating the suspension with a filter having a maximum pore size of ≤ 450 nm and preferably
d) reducing the water content to ≤ 95 weight-% relating to the total weight of the preparation and/or depleting the microbiota comprised in the suspension analogue to or by performing a second filtration with a maximum pore size of ≤ 20 nm.

The claimed IMEnT preparations (*e.g*., filtrates) and/or compositions and/or formulations and/or supplements are equally usable for the therapy or prophylaxis of diseases with similar genesis, *e.g*., necrotising enterocolitis, in animals, especially in both human and other mammals, in particular in domestic and useful animals. Examples of such animals are dogs, cats, cattle, horses, sheep, pigs, goats or camels without objective restriction. Donors and recipients are usually, but not necessarily, from the same species (Smits et al. 2013 Gastroenterology 145:946). For example, when using pig stool instead of human stool as the basis of the primary IMEnT preparation, the use of specific pathogen-free (SPF) minipigs under GMP conditions enables production of large, perfectly characterised and stable stool batches with a much better risk profile and manufacturing process and is therefore in many cases preferred (see Example 7).

As pathological changes in the intestinal microbiota can also play a causal role in numerous other diseases or disorders, the therapy and/or prophylaxis of such diseases or disorders is also within the scope of the invention. Indications in which IMEnT can be useful include, but are not limited to, intestinal inflammatory and/or infectious diseases, CDI and pseudomembraneous colitis resulting from *Clostridium difficile* toxins, other chronic gastrointestinal infections (e.g., by *Yersinia* spp., *Campylobacter* spp., *Aeromonas* spp., *Escherichia coli, Cryptosporidium* spp., *Shigella* spp., amoebae, Giardia, or chronic viral infections), small bowel bacterial overgrowth, IBD, Crohn's disease, ulcerative colitis, lymphocytic colitis, mucous colitis, collageneous colitis, microscopic colitis, antibiotic-associated colitis, enterohemorrhagic colitis, diverticular disease, AIDS enteropathy, irritable bowel syndrome, spastic colon, chronic diarrhea, idiopathic or simple or chronic constipation, eosinophilic disorders of the gastrointestinal tract, sacroiliitis, metabolic syndrome, obesity, diabetes, insulin resistance, non-alcoholic fatty liver disease, non-alcoholic steatohepatitis, cardiovascular diseases, arteriosclerosis, atherosclerosis, coronary heart disease, autoimmune diseases, atopic diseases, allergic diseases, atopic eczema, asthma, psoriasis, cystic fibrosis, COPD, systemic autoimmunity including rheumatoid arthritis and systemic lupus erythematosus, idiopathic thrombocytopenic purpura, chronic pain disorders such as fibromyalgia, chronic fatigue syndrome, eating disorders, malnutrition, and neurodevelopmental or neurodegenerative disorders, such as Parkinson's disease, multiple sclerosis or autism. As chronic intestinal inflammation strongly increases the risk of developing colon carcinoma (for review see *e.g*., Ullman & Itzkowitz 2011, Gastroenterology 140:1807), a use of IMEnT preparations and/or compositions and/or formulations and/or supplements according to the invention is also the prophylaxis of colon carcinoma in the case of a chronic or recurrent intestinal inflammation. For further indications for IMEnT treatment, see US 5443826 (column 2, line 26-65; column 3, line 26-57). In particular, IMEnT is useful to be administered before, during and/or after any systemic or topical antibiotic therapy affecting the intestinal microbiota in order to strengthen, protect, revitalise and/or normalise the intestinal microbiota of the subject treated with antibiotics.

Therefore, the present invention preferably pertains to a composition consisting of or comprising the preparation (*e.g*., the filtrate) as defined herein before and/or herein after as active ingredient, wherein the disease or disorder is selected from the group consisting of autoimmune, intestinal, metabolic, cardiovascular, atopic, allergic, neurodevelopmental, neurodegenerative, idiopathic and chronic pain diseases, preferably selected from the group consisting of intestinal inflammatory and/or infectious diseases, especially *Clostridium difficile* infection (CDI); pseudomembraneous colitis resulting from *Clostridium difficile* toxins; other chronic gastrointestinal infections *e.g.*, by *Yersinia* spp., *Campylobacter* spp., *Aeromonas* spp., *Escherichia coli, Cryptosporidium* spp., *Shigella* spp., amoebae, Giardia, or chronic viral infections; small bowel bacterial overgrowth, inflammatory bowel diseases (IBD), Crohn's disease, ulcerative colitis, lymphocytic colitis, mucous colitis, collageneous colitis, microscopic colitis, antibiotic-associated colitis, enterohemorrhagic colitis, diverticular disease, AIDS enteropathy, irritable bowel syndrome, spastic colon, chronic diarrhea, idiopathic or simple or chronic constipation, eosinophilic disorders of the gastrointestinal tract, sacroiliitis, metabolic syndrome, obesity, diabetes, insulin resistance, non-alcoholic fatty liver disease, non-alcoholic steatohepatitis, cardiovascular diseases, arteriosclerosis, atherosclerosis, coronary heart disease, autoimmune diseases, atopic diseases, allergic diseases, atopic eczema, asthma, psoriasis, cystic fibrosis, COPD, systemic autoimmunity including rheumatoid arthritis and systemic lupus erythematosus, idiopathic thrombocytopenic purpura, chronic pain disorders such as fibromyalgia, chronic fatigue syndrome, eating disorders, malnutrition, and neurodevelopmental or neurodegenerative disorders, such as Parkinson's disease, multiple sclerosis or autism.

The present invention also provides the use of preparations (*e.g*., filtrates) and/or compositions and/or formulations and/or supplements, *e.g*., a pharmaceutical composition or a composition suitable for dietary supplements, described herein for the manufacture of medicaments for treating or ameliorating or preventing one or more of the diseases, disorders or pathological or iatrogenic conditions described herein, and/or in the manufacture of dietary supplements for helping to prevent or ameliorate one or more of the diseases, disorders and conditions described herein.

Further, the present invention may provide the use of a preparation (*e.g.*, a filtrate) as defined herein before and/or herein after as active ingredient, for non-therapeutically positively influencing the intestinal microbiota of an animal, preferably of a mammal, in particular of a human.

The methodological elements for enabling the claimed invention are well known in the art. All techniques and methods mentioned and referred to in the present application are merely examples and shall not be construed as restrictions of any kind.

Exclusion criteria for FMT recipients (see, *e.g.*, Bakken et al. 2011 Clin. Gastroenterol. Hepatol. 9:1044, section III: "Recipient Exclusion Criteria", p. 1048) do not apply or do not have to be as stringent for IMEnT recipients, as the IMEnT preparations and/or formulations and/or compositions and/or supplements according to the present invention do not contain live microbiota.

For the same reason, the criteria for selecting IMEnT stool donors do not necessarily have to be as stringent as for FMT stool donors. These criteria may include and integrate, but are not limited to, the recommendations and guidelines described in the following literature:
- Bakken et al. 2011 Clin. Gastroenterol. Hepatol. 9:1044 (especially section II: "Donor Selection", pp. 1046-1048);
- Borody & Khoruts 2012 Nat. Rev. Gastroenterol. Hepatol. 9:88 (especially section "Guidance on FMT, p. 91);
- Hamilton et al. 2012 Am. J. Gastroenterol. 107:761 (especially section "Donor Identification and Screening", p. 763, and Supplementary Appendix 1);
- WO 2012/122478A1 (especially p. 12 line 10 to p. 13 line 32).

In a preferred embodiment, a well-characterised human stool donor population is set up in analogy to a blood donor population. The methods for setting up and maintaining such a population and for collecting samples are well known in the art (see, *e.g*., WO 2011/094027A1 in its entirety).

In a particularly preferred embodiment, pig stool instead of human stool is used for the primary IMEnT preparations to enable easier production of larger, more homogeneous and better characterisable batches without human risk factors, in particular when specific pathogen-free (SPF) minipigs are used under GMP conditions (see Example 7).

The starting material for IMEnT preparations can be fresh, cooled or frozen stool or stool preparations from culture or fermenting systems. Non-limiting examples for procedures to prepare stool are provided in the following literature:
- Hamilton et al. 2012 Am. J. Gastroenterol. 107:761 (especially section "Donor Material Preparation", p. 763);
- WO 2012/122478A1 (especially p. 5 line 5-18, p. 11 line 31 to p. 12 line 9 and p. 15 line 33 to p. 18 line 32);
- Youngster et al. 2014 Clin. Infect. Dis. 58:1515 (especially section "Preparation of Frozen Inocula", p. 1517).

Using stool pools and/or cultured and/or fermented stool and/or synthetic and/or artificial and/or fermented stool preparations as described herein for producing primary IMEnT preparations may be useful to increase batch sizes and, at the same time, homogeneity of the primary IMEnT preparations. In addition, pooling also decreases the risk of transferring unwanted contents from one or more stool samples. In one embodiment, stool can be cooled to just above the freezing point (*e.g.*, 4°C) and stored up to several days until processing. In another embodiment, stool can be cryopreserved using suitable cryoprotectants (see, *e.g*., WO 2012/122478A1 p. 11 line 31 to p. 12 line 9), as long as these are safe to administer to the IMEnT recipients. Pooling of stool samples or preparations can be performed at one or more stages throughout the production process. For example, fresh stool samples of two or more donors (*e.g*., humans or pigs) can be pooled, or IMEnT preparations from two or more stool donors can be pooled, or IMEnT preparations of two or more production batches can be pooled for the final formulation of an IMEnT composition. Until the separation of the microbiota from the primary IMEnT preparations, microbiota-conserving procedures are important also for IMEnT, because it is desirable to mirror the healthy intestinal microenvironment, and the stool used for IMEnT preparations shall not contain inappropriately large quantities of cell debris and/or substances released from dying or dead microbial cells due to inadequate environmental conditions. In culture or fermenting systems such as the large intestinal system (Minnekus *et al.* 1999 Appl. Microbiol. Biotechnol. 53:108) or the Robogut of the RePOOPulate system (Petrof et al. 2013 Microbiome 1:3; Petrof & Khoruts 2014 Gastroenterology 146:1573), the stool used for IMEnT preparations may be cultivated, e.g., in order to expand the microbiota and/or to selectively improve the microbiota by adding probiotics and/or prebiotics and/or synbiotics and/or an optimised standard stool from a well-characterised donor population as described herein to the stool culture and cultivating the resulting improved microbiota for appropriate periods. Moreover, artificial and/or synthetic stool, e.g. RePOOPulate (Allen-Vercoe & Petrof 2013 Expert Rev. Gastroenterol. Hepatol. 7:291; Petrof et al. 2013 Microbiome 1:3; WO 2013/037068 A1), may be produced by combining defined microbiota with or without fermentation and subsequently or continuously be used for the production of IMEnT preparations. In addition, detrimental ingredients may be removed from stool samples and/or stool cultures, e.g., as described herein.

The stool preparation protocols for IMEnT follow the guidelines for FMT protocols known in the art (see above) up to the point when a suspension of largely viable microbiota in water or a physiological solution or buffer, *e.g*., phosphate-buffered or physiological saline, has been produced. In a preferred embodiment, the physiological buffer for IMEnT preparations contains as few salt ions or other ingredients as possible, because these ingredients remain in the IMEnT preparations and/or compositions and/or formulations and/or supplements. In one of these preferred embodiments, the physiological buffer is physiological saline solution (0.9% w/v of sodium chloride in sterile water). From this point onwards, the focus of FMT techniques is on administering healthy microbiota in more or less concentrated form to the recipient, whereas the IMEnT protocol separates the liquid phase of the stool preparations from the microbiota (preferably also from removable enteric viruses) and prepares diverse compositions and/or formulations and/or supplements from this liquid primary IMEnT preparations.

Techniques to separate microbiota like bacteria and fungi from the liquid or medium in which they have grown or been prepared are well known in the art of the pharmaceutical and/or the food industry. These techniques include, *e.g*., centrifugation and/or dead-end filtration for smaller volumes, or cross-flow/tangential flow filtration/diafiltration for larger volumes. For all separation (*e.g*., filtration) techniques, it is known by the person skilled in the art that separators (*e.g*., filters) do not lead to 100% removal of undesirable particles of a given size (*e.g*., microbiota) from a solution, but that the efficacy of separation (*e.g*., filtration) is measured by log-scale reduction (*e.g*., of microbiota) and validated by loading filters with defined amounts of defined test organisms. For depletions according to the present invention, the filter (maximum) pore size must be approximately ≤ 450 nm to ensure that the filtrate is depleted of microbiota. Such a pore size will remove all eukaryotic and practically all prokaryotic cells (bacteria and archaea). It is preferred that the (maximum) pore size is approximately ≤ 220 nm (for historical reasons, this size has long been used for sterile filtration by certain filter manufacturers) or ≤ 200 nm (another standard for sterile filtration used by certain filter manufacturers). It is further preferred that the (maximum) (analogue) pore size is approximately ≤ 100 nm. A depletion according to those sizes will result in a primary IMEnT preparation or a preferred primary IMEnT preparation (for maximum pore sizes ≤ 450 nm).

In one embodiment, the resulting primary IMEnT preparation may be subsequently depleted from removable enteric viruses, which may or may not be contained in the primary IMEnT preparation, by dead-end or cross-flow/tangential flow filtration or other techniques suitable to offer a pore size of approximately ≤ 20 nm (e.g., using Pegasus SV4 filters from Pall, Virosart HC filters from Sartorius Stedim Biotech, or Planova 20N or 15N filters from Asahi-Kasei Bioprocess). The preparation of liquid, microbiota-depleted stool specimens (e.g., by filtration or by centrifugation) is already used in art for a completely different purpose, namely for analysis of free intestinal viruses or cell-based assays for stool water components, e.g., for cytotoxin assays to test for the presence or concentration of *Clostridium difficile* toxin(s). However, the use of microbiota-depleted stool preparations (the primary IMEnT preparations) in pharmaceutical compositions and/or formulations and/or dietary supplements as presented in the present invention has not been described in the available literature. Enteric virus removal, *e.g.*, by coal-based media, is being used in water purification devices and plants. However, a virus removal step as used in the present invention has not been described for stool preparations in the available literature. With or without a virus removal step, the resulting liquid IMEnT preparations can be further processed into diverse compositions and/or formulations and/or supplements. In addition, sterilisation (*i.e.*, rendering microbiota and/or viruses harmless without removing them from the primary or secondary IMEnT preparations) of the stool preparations by autoclaving, irradiation or ultrasound treatment is also within the scope of the present invention.

In another embodiment, the resulting primary or secondary IMEnT preparations may be fractionated to prepare certain active agent fractions like, e.g., antimicrobial peptides, oligonucleotides (e.g., those smaller than 30 base pairs or bases), bile acids, bile acid derivatives (e.g., secondary bile acids) or fatty acids (e.g., short chain fatty acids). Such substances, substance groups and/or fractions may also be artificially and/or synthetically be produced and used for all purposes according to the present invention. Moreover, IMEnT preparations may also be enriched in such substances, substance groups and/or fractions, *e.g.*, by adding such substances, substance groups and/or fractions either derived from IMEnT preparations or from synthesis or other sources.

The preparations and/or compositions and/or formulations and/or supplements of the present invention may be included in a diversity of pharmaceutically acceptable formulations including diverse pharmaceutical carriers and/or excipients, as described, for example, in WO 2012/122478A1 (p. 13 line 33 to p. 15 line 32), or in formulations acceptable for dietary supplements.

It can be preferred that the liquid primary or secondary IMEnT preparations are formulated for liquid storage and application via nasal (nasogastric or nasointestinal) or rectal routes of administration. Such preparations may be concentrated (e.g., to reduce storage volume) and/or (further) expanded to a final application volume before use.

It can be preferred that the liquid primary or secondary IMEnT preparations are freeze-dried, lyophilised or spray-dried according to methods known in the art of the pharmaceutical and/or the food industry to protect as many of their components as possible from changing or degrading and to provide dry secondary IMEnT preparations which can be easily stored, processed and/or mixed with other components for a final formulation.

It can be preferred that such dry secondary IMEnT preparations can be rehydrated before application via nasal (nasogastric or nasointestinal) or rectal routes of administration.

It can be preferred that such dry secondary IMEnT preparations are incorporated in oral or rectal dosage forms for administration as a medicament or nutritional supplement (for details of preferred formulations and delivery forms, see below).

It can be preferred that such liquid primary or secondary IMEnT preparations are frozen with or without prior concentration, with or without addition of cryoprotectants or other additives or excipients. From such frozen and/or concentrated and/or formulated primary or secondary IMEnT preparations, an IMEnT working solution for nasal (nasogastric or nasointestinal) or rectal administration may be produced by thawing and/or diluting the liquid IMEnT preparation.

It also can be preferred that highly concentrated liquid secondary IMEnT preparations are prepared and formulated for oral delivery. For example, such highly concentrated IMEnT solutions can be formulated with gelling agents with or without other excipients and filled in capsules as a gel or viscous liquid. Based on combination therapies with already known active substances or compositions, *e.g.*, probiotics, prebiotics, synbiotics, proteins and/or enzymes supporting probiotics; antibiotics, antimycotic, antiprotozoal, antihelminthic, antiviral or anti-inflammatory agents; aminosalicylates; nicotinic acid; nicotinamide; tryptophan; compounds that convert in the body of an animal or human into nicotinic acid, nicotinamide or tryptophan; nicotinamide adenine dinucleotide (NAD); nicotinamide adenine dinucleotide phosphate (NADP); intermediates in the biosynthesis of NAD or NADP; tryptophan dipeptides; short-chain fatty acids (SCFA); medium-chain fatty acids (MCFA); systemic or topical corticosteroids; β₂-adrenergic receptor agonists; theophylline and other substances of the xanthine family; statins; small molecules; peptides and biologicals, IMEnT preparations may be even more suited for treating the above-mentioned diseases. On the one hand, the combination can consist of offering a single pharmaceutical composition which contains IMEnT preparations in combination with one or more of the below active substances or compositions, or the patient is given several preparations containing one or more of these active substances or compositions simultaneously with, or time-staggered with respect to, a pharmaceutical IMEnT composition or an IMEnT nutritional supplement according to the invention.

As used herein, the term "antibiotic" denotes an antibacterial agent that inhibits bacterial growth or kills bacteria. The term "antibacterial" is often used synonymously with the term antibiotic(s). In its broadest sense the term "antibiotic" may include efficacy against the causative agents of one or more infectious diseases, and "antibiotic" then denotes a broader range of antimicrobial compounds, including anti-fungal and other compounds.

As used herein, the term "antimycotic agent" denotes the property of a substance or treatment to cure or control mycotic infections.

As used herein, the term "antiprotozoal agent" denotes the property of a substance or treatment to cure or control protozoal infections.

As used herein, the term "antihelminthic agent" denotes the property of a substance or treatment to cure or control helminthic infections.

As used herein, the term "antiviral agent" denotes the property of a substance or treatment to cure or control viral infections.

As used herein, the term "anti-inflammatory agent" denotes the property of a substance or treatment to reduce inflammation.

As used herein, the term "short-chain fatty acid" (abbreviated SCFA) denotes a subgroup of linear or branched fatty acids with aliphatic tails of two to six carbons.

As used herein, the term "medium-chain fatty acid" (abbreviated MCFA) denotes a subgroup of linear or branched fatty acids with aliphatic tails of six to 12 carbons.

As used herein, the term "small molecule" denotes an active ingredient of a medicinal drug or a dietary supplement, which has a low molecular weight (≤ 1,000 Da), and a preferably chemically manufactured organic compound that influences biological processes. However, "small molecule" also denotes compounds of natural origin.

As used herein, the term "peptide" denotes a compound containing two or more amino acids and preferably ≤ 50, more preferably ≤ 20 amino acids, in which the carboxyl group of one amino acid is linked to the amino group of the other via a peptide bond.

As opposed to small molecules, the term "biological" denotes an active ingredient of a medicinal drug (biomolecular drug) or a dietary supplement, which is produced by biological processes and is larger than 1,000 Da. Examples of biologicals are antibodies, fusion proteins and larger peptides (which are not or cannot be produced by chemical synthesis).

Preferably, IMEnT preparations or compositions are combined with one or more active ingredients of, e.g., the said probiotics, prebiotics or synbiotics, proteins and/or enzymes supporting probiotics, antibiotics, antimycotic agents, antiprotozoal agents, antihelminthic agents, antiviral agents, anti-inflammatory agents, small molecules, peptides and/or biologicals, or other substance classes which are effective against the diseases or disorders mentioned above, such as
- probiotics as defined above;
- prebiotics as defined above;
- synbiotics as defined above;
- proteins and/or enzymes supporting probiotics;
- antibiotics, including bactericidal and bacteriostatic antibiotics targeting specific types of bacteria, such as Gram-negative or Gram-positive bacteria, as well as broad-spectrum antibiotics affecting a wide range of bacteria. Examples of antibiotics include, but are not limited to: inhibitors of the cell wall synthesis (betalactam antibiotics such as penicillins, cephalosporins, carbapenems and monobactams; glycopeptides such as vancomycin or teicoplanin; phosphomycin); antibiotics that target the cell membrane like polymyxines or lipopeptides; folic acid synthase inhibitors, such as sulphonamides or diaminopyrimidines; DNA-targeting antibiotics, such as fluoroquinolones, ansamycins, macrocyclins (e.g., fidaxomicin), nitroimidazoles or nitrofuranes; inhibitors of bacterial protein synthesis, such as oxazolidinones, aminoglycosides, tetracyclins, glycylcyclines, macrolides, lincosamides or streptogramins; antituberculosis agents like isoniazid, ethambutol, pyrazinamid, delaminid or bedaquiline; fusidic acid; chloroamphenicol; rifamycins; lipiarmycins;
- antimycotic agents, including, but not limited to, ergosterol synthesis inhibitors (e.g., allylamines, azoles, morpholines or thiocarbamates); polyenes; flucytosine; griseofulvin; echinocandins; ciclopirox;
- antiprotozoal agents, including, but not limited to, metronidazole; suramin; pentamidine; nifurtimox; benznidazole; miltefosine; sodium antimony gluconate; meglumin antimoniate; paronomycin; sulfadiazine; pyrimethamine; folinic acid; spiramycin; quinine; chloroquine; mefloquine; proguanil; atovaquone; artemisinine and its derivatives; lumefantrine; doxycyclin;
- antihelminthic agents, including, but not limited to, praziquantel; niclosamide; mebendazole; albendazole; pyrantel embonate; pyrvinium embonate;
- antiviral agents, including, but not limited to, those directed against viruses causing or contributing to intestinal pathologies and/or symptoms, *e.g.*, antiinfluenza compounds such as amantadine, oseltamivir or zanamivir; inhibitors of viral DNA or RNA polymerases, such as acyclovir, valaciclovir, penciclovir, famciclovir, ganciclovir, valganciclovir, brivudine, trifluridine, foscarnet or ribavirine; anti-HIV agents like nucleoside or non-nucleoside reverse transcriptase inhibitors, protease inhibitors, fusion inhibitors or integrase inhibitors; agents directed the hepatitis B virus, *e.g*., interferon-a or nucleoside reverse transcriptase inhibitors; agents directed the hepatitis C virus, *e.g*., interferon-a, RNAse inhibitors like ribavirine, or protease inhibitors;
- aminosalicylates, *e.g.*, sulfasalazine, mesalamine, olsalazine, balsalazide;
- active substances selected from nicotinic acid; nicotinamide; tryptophan; a compound that converts in the body of an animal or human into nicotinic acid, nicotinamide or tryptophan; nicotinamide adenine dinucleotide (NAD); nicotinamide adenine dinucleotide phosphate (NADP); an intermediate in the biosynthesis of NAD or NADP; a tryptophan dipeptide; or a combination thereof;
- short-chain fatty acids, *e.g.*, propionate, butyrate, and/or their esters;
- medium-chain fatty acids, *e.g*., caproate, caprylate, caprate, laurate and/or their esters;
- systemic or topical corticosteroids, *e.g*., glucocorticoids such as cortisone, hydrocortisone, prednisone, prednisolone, methylprednisolone, dexamethasone, betamethasone, triamcinolone or budenoside, or mineralcorticoids, such as aldosterone or fludrocortisone;
- β₂-adrenergic receptor agonists, *e.g.*, salbutamol, levosalbutamol, terbutaline, salmeterol or formoterol;
- theophylline and other substances of the xanthine family;
- statins, *e.g.*, atorvastatin, cerivastatin, fluvastatin, lovastatin, mevastatin, pitavastatin, pravastatin, rosuvastatin or, preferably, simvastatin;
- peptides, *e.g*. gut peptides and/or gastrointestinal hormones;
- biologics comprising (monoclonal) antibodies or antibody derivatives, *e.g.*, antitumour necrosis factor antibodies like infliximab, adalimumab, golimumab, certolizumab; or other antibodies like tocilizumab, natalizumab, vedolizumab, ustekinumab, rituximab, trastuzumab, alemtuzumab, denosumab, panitumumab, eculizumab, cetuximab or bevacizumab;
- biologics comprising fusion proteins or peptides, *e.g*., etanercept, abatacept, belatacept, aflibercept, rilonacept, romiplostim, or alefacept.

A combination may be present in the same or in separate dosage forms, which may be administered simultaneously or sequentially, and/or it may combine one or more active ingredients with IMEnT in a fixed or variable dose combination.

As used herein, the term "variable dose combination" refers to a combination of two or more active substances in drugs or dietary supplements, whereby each of these substances is applied in the form of a separate composition, *e.g*., two single dosage forms, which separate composition may be administered together by a consecutive or subsequent administration regimen. For example, a composition of a probiotic in any suitable dosage thereof may be administered together, consecutively or subsequently, with a separate IMEnT composition in any suitable dosage thereof. Thus, variable dosages of one or more active substances, *e.g*., of a probiotic and/or a prebiotic, may be combined with variable dosages of an IMEnT preparation. These variable dose combinations may use conventionally available compositions of active ingredients as exemplified above or may be also achieved by customized polypharmacy via compounding.

In contrast to a variable dose combination, a fixed-dose combination is a combination drug or dietary supplement, which is a formulation including two or more active ingredients combined in a single dosage form, which is manufactured and distributed in certain respective fixed doses. A fixed-dose combination mostly refers to a mass-produced product having a predetermined combination of drugs or dietary supplements (active substances) and respective dosages.

Thus, the invention also pertains to embodiments of a composition or the use thereof as described herein, wherein the composition is a controlled and/or delayed release IMEnT formulation, or a variable dose combination or a fixed dose combination of a controlled and/or delayed release IMEnT formulation with a formulation of other active ingredients as exemplified in the present invention.

For the treatment of CDI, e.g., for acute or chronic CDI or for the prevention of CDI relapses, combinations of IMEnT preparations and/or compositions and/or formulations and/or supplements with one or more of the following agents are preferred: metronidazole; vancomycin; fidaxomicin; rifaximin; teicoplanin; fusidic acid; nitazoxanide; rifampin; bacitracin; (intraveneous) immunoglobulins, monoclonal antibodies, single-domain antibodies, antibody derivatives, fusion proteins and/or combinations of such proteins directed against *Clostridium difficile* and/or *Clostridium difficile* toxins [examples include, but are not limited to, the anti-toxin antibodies 3H2, CDA1, 1B11, MDX-1388, 103-174, 1G10, and 2A11 (Babcock et al. 2006 Infect. Immun. 74:6339), PA-50 and PA-41 (Marozsan et al. 2010 J. Infect. Dis. 206:706) MK3415 and MK6072 (Koon et al. 2013 Antimicrob. Agents Chemother. 57:3214) and/or CDA1 and CDB1 (Lowy et al. 2010 N. Engl. J. Med. 362:197)]; anion binding resins such as colestipol, cholestyramine and/or tolevamer; probiotics, prebiotics and/or synbiotics; proteins and/or enzymes supporting probiotics.

Such combination therapies can serve the purpose of not allowing acute disease conditions to become chronic, as the IMEnT preparations and/or compositions and/or formulations and/or supplements according to the invention and the other active ingredients counteract complementary aspects of the pathophysiological mechanisms underlying the disease. In combination with glucocorticoids, for example, one positive effect is a result of the circumstance that fewer glucocorticoids may have to be used so as to achieve a savings effect and reduce, or even prevent, the side-effects known for glucocorticoids. Similar beneficial aspects probably apply for the combination use of IMEnT with antibiotics.

In a preferred embodiment of the present invention, IMEnT preparations and/or compositions and/or formulations and/or supplements are combined with one or more other active ingredients or formulated drugs with slow, delayed or retarded topical release and/or with systemic drugs in order to support the topical efficacy (*e.g*., the reduction of inflammation in the intestinal mucosa) and/or to support the intestinal uptake of these ingredients or drugs by partly or completely normalising the intestinal flora. Preferably, IMEnT preparations and/or compositions and/or formulations and/or supplements are combined with one or more topical agents selected from the group consisting of antibiotics; antimycotic, antiprotozoal, antihelminthic, antiviral or anti-inflammatory agents; aminosalicylates; nicotinic acid; nicotinamide; tryptophan; compounds that converts in the body of an animal or human into nicotinic acid, nicotinamide or tryptophan; nicotinamide adenine dinucleotide (NAD); nicotinamide adenine dinucleotide phosphate (NADP); intermediates in the biosynthesis of NAD or NADP; tryptophan dipeptides; short-chain fatty acids; medium-chain fatty acids; corticosteroids; β₂-adrenergic receptor agonists; theophylline and other substances of the xanthine family; statins; small molecules; peptides and biologicals.

As used herein, the term "topical efficacy" refers to a topical effect, in the pharmacodynamic sense, and thus refers to a local, rather than systemic, target for a medication. "Topical" is defined in the art as relating or being applied to a part of the body (Oxford dictionary), e.g., the skin or the intestinal epithelium, as opposed to "systemic" actions relating to distribution via the circulatory system. Accordingly, topical or local efficacy means a local therapy and/or prophylaxis of an active substance specifically or selectively to a location where, for example, the medication or dietary supplement or food ingredient shall deliver its direct therapeutic and/or prophylactic effect.

For oral administration, the primary or secondary IMEnT preparations are preferably used in a pharmacological or dietary supplement formulation that protects the largest possible amount of active substances from being absorbed by the body in the stomach or upper small intestine and rather effects a release (*e.g*., controlled release and/or delayed release) into the small intestine and/or colon where the intestinal microbiota to be modified are located (*e.g.*, the active substances are selectively released in the small intestine and/or colon).

In particular, the active substances of IMEnT preparations as described herein are thus suitable for being used in medicaments or dietary supplements with topical release (*e.g*., controlled and/or delayed release) for the therapy and/or prophylaxis of the indications mentioned above.

Thus, the present invention preferably pertains to a composition and/or formulation and/or supplement consisting of or comprising secondary IMEnT preparations as defined herein before and/or herein after as active ingredient, wherein the composition is for oral administration, more preferably with controlled and/or delayed release, of the active ingredients for specific local effectiveness in the small and/or large intestine, further preferably in the lower small intestine and/or colon.

As used herein, the "lower small intestine" is the second half of the small intestine.

As an alternative, the present invention preferably pertains to a composition and/or formulation and/or supplement consisting of or comprising the preparation as defined herein before and/or herein after as active ingredient, wherein the composition is for (neo)rectal administration in the colon or pouch.

As a further alternative, the present invention preferably pertains to a composition and/or formulation consisting of or comprising the preparation as defined herein before and/or herein after as active ingredient, wherein the composition and/or formulation is for nasal administration. Nasal admimistration routes include, *e.g*., the administration via nasogastric, nasointestinal, nasoduodenal, nasojejunal or nasoileal tube infusion.

Pharmaceutical compositions and/or dietary supplements which contain primary or secondary IMEnT preparations can be administered orally with a controlled and/or delayed (retarded) active substance release. In addition, said pharmaceutical compositions can also be administered rectally (*e.g.*, via enemas or suppositories). The site of delivery of the active IMEnT substances is preferably the lower small intestine and/or the colon.

In order to produce orally administered formulations of secondary IMEnT preparations having a beneficially modifying and/or anti-inflammatory effect on the intestinal microbiota in the small (preferably in the lower small) intestine and/or in the colon, it is thus advantageous and innovative to use controlled and/or delayed modes of release.

In order to treat, *e.g*., CDI, Crohn's disease or ulcerative colitis, nasal, oral and/or rectal modes (*e.g*., as enema) of application are suitable. In order to treat pouchitis in the case of ulcerative colitis, the rectal application (*e.g.,* as enema) is preferred. It can also be supported by an oral administration of the oral formulations described above, *e.g*., controlled and/or delayed releasing preparations. For the symptomatic therapy of any other form of colitis, nasal, oral and/or rectal applications can be chosen for the therapeutic modification of the intestinal microbiota. The oral application is preferred for the prophylaxis of the colon carcinoma, in particular in the case of ulcerative colitis, and for the therapy and/or prophylaxis of other diseases, which partially or substantially result from changes in the intestinal microbiota and/or an impaired interaction between intestinal microbiota and the intestines and in which nasal delivery is not an option or not preferable.

For oral administration, particular dosage forms that control and/or delay the release of the active substance due to special galenics (so-called controlled release, slow release, retarded release or delayed release forms) are particularly suitable. Such dosage forms may be simple, coated or uncoated capsules or tablets, *e.g*., coated capsules, film tablets or dragees. The tablets are usually round or biconvex. Oblong tablet forms, which allow the tablet to be separated, are also possible. In addition, granules, spheroids, pellets or microcapsules are preferred, which are filled in sachets or capsules, where appropriate. In a further embodiment, preformed tablets or similar solid, compacted dosage forms may also be prepared in shapes tightly fitting into capsules or microcapsules. These solid inserts can then be encapsulated and thus innovatively combine the advantage of higher active substance content (more dry secondary IMEnT preparation in a smaller space compared to filling capsules with, *e.g.*, non-compacted IMEnT lyophilisate) with the advantages of a capsule (*e.g*., stability and reliable containment of odor or taste of the IMEnT preparation, which is less unpleasant than that of fresh stool preparations, but still uncomfortable for the patient or consumer; see Examples below). In yet another embodiment of the present invention, capsules can be filled with gels or viscous concentrates of IMEnT preparations (see above).

The term "delayed release" relates preferably to a formulation that releases the active ingredients after a period of delay. In certain embodiments, the delay is sufficient for at least a portion of the active substances in a formulation to release in the small intestine (preferably, in the lower small intestine, *e.g*., in the terminal ileum) and/or colon.

The term "controlled release" refers preferably to a formulation or component thereof that releases, or delivers, one or more active ingredients over a prolonged period of time (time-dependent release) and/or under certain physiological conditions (*e.g*., pH-dependent release). In certain embodiments, the period of time or the release according to physiological conditions (*e*.*g*., pH) is sufficient for at least a portion of the active substances in a formulation to release in the small intestine (preferably in the lower small intestine, *e.g.*, in the terminal ileum) and/or colon.

The retardation and/or delayed release and/or controlled release is advantageously achieved, *e.g.*, by coatings which are resistant to gastric juice and dissolve depending on the pH, by means of microcellulose and/or multi matrix (MMX) technologies, by using different carrier matrices or a combination of these techniques. Examples include film coatings which contain acrylic and/or methacrylate polymers in various mixtures for controlled and/or delayed release. Additional examples include biodegradable polymers like natural or chemically modified pectins as polymer-drug conjugates, coatings and/or matrix agents for microbiota-dependent release (reviewed, e.g., by Vandamme et al. 2002, Carbohydrate Polymers 48:219). For example, the active IMEnT substances can be contained in a conventional matrix of microcrystalline cellulose or gelatin or with MMX technology, which can be coated with a material which provides the delayed and/or controlled release of the active IMEnT substances. It is preferred to introduce an active IMEnT substance in capsules [*e.g.*, gelatin or hydroxypropylmethyl cellulose (HPMC) capsules or microcapsules], which may be coated by means of known methods. If the content of the capsule shall only be protected from stomach acid and delivered to the small intestine, gelatin or HPMC (micro)capsules of diverse sizes and properties can be sufficient to ensure delivery to the small intestine as well taste and odor masking [see, *e.g*., the complete product portfolio of Capsugel (Morristown, NJ, USA)]. In addition to capsules, many other options for taste and odor masking of mono- or multiparticulate formulations (such as pellets, granules etc.) can be used for the present invention, *e.g*., the lipid multiparticulate technology (Capsugel), the Eudragit^{®} E PO coating (Evonik Industries AG, Essen, Germany) or the Marcoat^{™} technology (Emerson Resources, Inc., Norristown, PA, USA). In addition, the core of pellets, granules, microcapsules and the like may be formulated from compressed IMEnT preparations in order to deliver more active IMEnT substances in a small volume.

Suitable coating agents for controlled and/or delayed release comprise water-insoluble waxes, such as carnauba wax, and/or polymers, such as poly(meth)acrylates [*e.g*., the poly(meth)acrylate product portfolio with the trade name Eudragit^{®}, in particular Eudragit^{®} L 30 D-55 (an aqueous dispersion of anionic polymers with methacrylic acid as a functional group), Eudragit^{®} L 100-55 (which contains an anionic copolymer based on methacrylic acid and ethyl acrylate), Eudragit^{®} L 100 or L 12,5 or S 100 or S 12,5 (anionic copolymers based on methacrylic acid and methyl methacrylate), or Eudragit^{®} FS 30 D (an aqueous dispersion of an anionic copolymer based on methyl acrylate, methyl methacrylate and methacrylic acid); Evonik Industries AG, Essen, Germany] and water-insoluble celluloses (*e.g*., methyl cellulose, ethyl cellulose). Where appropriate, water soluble polymers *(e.g.,* polyvinylpyrrolidone), water-soluble celluloses (*e.g.,* hydroxypropylmethyl cellulose or hydroxypropyl cellulose), emulsifiers and stabilisers (e.g., polysorbate 80), polyethylene glycol (PEG), lactose or mannitol can also be contained in the coating material.

For example, a combination of Eudragit^{®} S and L compounds (e.g., Eudragit^{®} L/S 100) effects a controlled release of the active substances according to the invention at pH > 6.4, which occurs in the terminal ileum. Further uses of Eudragit^{®} preparations and mixtures thereof (FS, L, S and R compounds) are also conceivable for the packaging of an active substance, and therefore a topical use in selected portions of the entire gastrointestinal tract can be achieved by controlled release at certain pH values. A systematic study of enteric targeting with hydroxypropyl methylcellulose (HPMC) capsules and more recently developed Eudragit^{®} polymers was published by Cole et al. in 2002 (Int. J. Pharm. 231:83).

Non-limiting examples especially for the formulation of dietary supplements, but also food ingredients, according to the present invention have been described recently by Berg et al. (2012, J. Food Eng. 108:158) for highly water soluble substances. Thus, the active substances according to the present invention can, *e.g.*, be formulated as lyophilised or spray-dried maltodextrin-pectin microcapsules and shellac-coated granulates.

The pharmaceutical composition can also contain further pharmaceutical excipient substances, such as binders, fillers, glidants, gelling agents, lubricants and flow regulating agents. The compounds according to the invention can be formulated, where appropriate, together with further active substances and with excipients conventional in pharmaceutical compositions, *e*.*g*., talcum, gum arabic, lactose, starch, magnesium stearate, cocoa butter, aqueous and non-aqueous carriers, lipid components of animal or vegetable origin, paraffin derivatives, glycols (in particular polyethylene glycol), various plasticizers, dispersants, emulsifiers, gelling agents and/or preservatives.

In order to produce enemas or suppositories for rectal application, preparations of active IMEnT substances can be dissolved in a suitable solvent and be further processed into enemas or suppositories according to known pharmaceutical methods.

The active IMEnT substance content in the finished dosage form is 1 ― 3000 mg, preferably 100 ― 1000 mg, in the case of oral administration; the formulations for nasal or rectal delivery can contain an amount of 10 ― 5000 mg of the active IMEnT substances. Depending on the intensity and severity of the disease or condition, the dosage forms are administered once or several times daily or, in the case of medicinal formulations, in another dosage regimen to be chosen by a physician.

As used herein, the terms "treatment", "treat" and "treating" refer to reversing, alleviating, delaying the onset of, or inhibiting the progress of a disease or disorder, or one or more symptoms thereof, as described herein. In some embodiments, treatment may be administered after one or more symptoms have developed. In other embodiments, treatment may be administered in the absence of symptoms. For example, treatment may be administered to a susceptible individual prior to the onset of symptoms (*e.g.*, in light of a history of symptoms and/or in light of genetic or other susceptibility factors). Treatment may also be continued after symptoms have resolved, for example to prevent or delay their recurrence.

As used herein, the terms "prophylaxis" and "prevent" refer to delaying the onset of or reducing the likelihood of developing a disease or disorder or one or more symptoms thereof, as compared to an untreated control population.

As used herein, the term "non-therapeutic" is opposed to "therapeutic", which denotes a use for treatment or prophylaxis as defined above. Non-therapeutic uses of the embodiments of the present invention include, but are not limited to, oral ingestion of dietary supplements containing IMEnT preparations or their derivatives with the aim of enhancing the general well-being by keeping the intestinal microbiota in good shape, *e.g.*, as IMEnT-alone formulations or by combining IMEnT with pro-, pre- oder synbiotics. Many consumers are healthy in terms of not having a specific disease, but wish to feel more comfortable about their intestinal health and their nutrition. Using IMEnT in such scenarios is by definition not a therapy or prophylaxis (in the absence of a disease or a clear prognostic disease marker), but rather an example of non-therapeutic use.

A further aspect of the invention described herein is the efficient use of the claimed medicaments and/or dietary supplements on the basis of genetic and/or microbiological data and specific needs of the individuals to be treated. New insights into the genetic predisposition of individuals for all types of diseases (in particular also diseases where the interaction between intestinal microbiota and intestines is impaired) and into pharmacogenetics indicate that an evidence-based personalised medicine including genetic analyses of relevant risk genes and also of genes which code *e*.*g*., for cell surface receptors, transporter proteins, metabolism enzymes or signal transduction proteins, which interact with the medicament and/or dietary supplement and/or its metabolites and/or its downstream effectors, can contribute information and improvements with respect to the type of use, the mode of application, the time(s) of use, the dose and/or the dosage regimen of the medicaments and/or dietary supplements described herein. This applies especially to analyses of the intestinal microbiota, particularly when a stool sample indicates a change in the microbiota. The present invention thus also comprises the use of suitable genetic and/or microbiological test methods to identify individuals particularly susceptible to the medicaments and/or dietary supplements according to the invention and/or to adapt the use of the medicaments and/or dietary supplements according to the invention to the individual circumstances. This also comprises expressly the use of different IMEnT preparations in different modes of administration depending on the genetic and microbiological properties of the individual. For these purposes, it is possible to use laboratory tests and/or suitable test kits and also measuring methods, devices and/or kits to be employed by a physician, user and/or patient, *e.g*., to take stool samples or to analyze suitable parameters in the blood, urine or other body fluids.

In addition, the present invention also relates to using the intestinal microbiota in part and/or in their entirety (the microbiome) as biomarkers to identify beneficial microbiota and/or detrimental microbiota, to support patient or subject selection for the treatments or preventions or uses described herein, to personalise and adapt the pharmaceutical compositions and/or treatments and/or preventions and/or dietary supplements described herein, and/or to determine end points and efficacy benchmarks for the pharmaceutical compositions and/or treatments and/or preventions and/or uses described herein.

Further methods and treatments can include the examples given in WO 2012/122478A1 (p. 18 line 33 to p. 20 line 31).

### EXEMPLIFICATION

There are variable possibilities to advantageously develop, and develop further, the teaching of the present invention. For this purpose, reference is made to the examples below which describe the invention in a representative way. It will be clear that the invention can be practiced otherwise than as particularly described in the foregoing description and in the following examples.

### Example 1:

Fresh stool from an individual of a human donor population meeting the donor inclusion criteria according to the invention is collected. Approximately 10 g each of stool are transferred to 2 sterile 50-mL centrifugation tubes and physiological saline (0.9% w/v of sodium chloride in sterile water) solution is added to a total volume of 45―50 mL. The stool is suspended and homogenised by vortexing or intensive shaking of the tubes. The resulting homogenate is filtered twice through cellulose filter paper with a pore size of approximately 10 µm or less to remove larger particles. The pre-filtered suspension containing the microbiota is then centrifuged at approximately 8,000 xg for 15 min at 4°C to pellet most of the microbiota. Subsequently, the supernatant is sterile-filtered through a 0.2 µm filter (Millex^{®}, Merck). For virus reduction or removal, the filtrate is then further processed using Pegasus SV4 filters from Pall according to the manufacturer's recommendations. Alternative filtration procedures after removing the larger particles are, in brief, ultracentrifugation (16,000 xg) for 20 min at 4°C or using a PDH4 depth filter (Pall), followed by sterile filtration of the supernatant via a Supor^{®} EKV filter (Pall; first layer: 1.2 to 0.2 µm, second layer: 0.2 µm) and virus filtration using a Pegasus^{™} SV4 (20 nm) filter. The resulting primary IMEnT preparation is applied to the IMEnT recipient via nasointestinal tube.

### Example 2:

On a given production day for IMEnT preparations, fresh stool from one or more individuals of a donor population meeting the donor inclusion criteria according to the invention is collected and stored airtight and cooled, preferably to approximately 4°C, until all stool donations scheduled for the day have been obtained. The collected stool is pooled and homogenously dispersed in physiological saline solution (0.9% w/v of sodium chloride in sterile water). The resulting stool homogenate is filtered through disposable filters of decreasing pore size and finally cross-flow filtered through a 0.2 µm filter in order to produce a sterile filtrate. For virus reduction or removal, the filtrate is then further processed using Pegasus SV4 filters from Pall according to the manufacturer's recommendations. The resulting primary IMEnT preparation is spray-dried, and the resulting dry secondary IMEnT preparation is encapsulated in large-volume capsules with a coat of Eudragit^{®} L/S 100 for release in the terminal ileum.

### Example 3:

Fresh stool from volunteers from a pre-selected healthy donor population meeting the donor inclusion criteria according to the invention was collected and stored in an airtight container at 4°C until processing. As a general rule, stool was processed no later than 2 h after collection, and the transplantation to the recipient was performed no later than 6 h after stool donation.

In a dedicated biosafety cabinet, approximately 50 g of fecal material was weighed and transferred to a standard commercial blender (Moulinex Multi Moulinette 400 W; SEB, Écully, France). Subsequently, 500 ml of sterile normal saline (0.9% sodium chloride) were added and the stool was homogenised for approximately 1 min at the highest level. The resulting slurry was then distributed into 10 50-ml centrifugation tubes (Sarstedt; Nümbrecht, Germany) and centrifuged for 10 min at approximately 1,800 xg to pellet large particles. The resulting supernatant was transferred to a beaker and filtered three times through disposable cellulose paper filters (pore size: 5―10 µm) placed in a stainless-steel sieve. According to the state of the art in FMT, the resulting pre-filtered slurry containing the fecal microbiota would now be applied to the recipient by nasointestinal tube.

According to the present invention, the pre-filtered slurry was then further filtered using a custom-built air pressure filtration unit (PALL; Dreieich, Germany) including a 5.7 L (6 bar) UCON pressure unit (UCON; Hausach, Germany) and a WIKA 1/2" TC inline diaphragm seal (―/+6 bar, S/N; WIKA; Klingenberg, Germany) at a pressure of 1.5―2 bar and the following steps:
1. small particle removal using two consecutive depth filters [1. filter: Seitz K 700 P 60 D (retention rating: 6.0-15.0 µm; PALL); 2. filter: Seitz KS 50 P 60 D (retention rating: 0.4-0.8 µm; PALL)];
2. microbiota depletion using a SUPOR EKV Filter Mini Kleenpak 0,2 µm unit (PALL).

The resulting primary IMEnT preparation was a light brown, clear liquid with a significantly less unpleasant and intensive odor compared to the pre-filtered, microbiota-rich slurry used in conventional stool preparations for FMT. This primary IMEnT preparation was transferred to a plastic bag (APPLIX HydroBag; Fresenius KABI; Bad Homburg, Germany) and applied to the recipient (patient) via a nasojejunal tube inserted by gastroscopy and controlled by an APPLIX Smart/Vision system (Fresenius KABI) within approximately 30 min. Before administering the IMEnT preparation, 1,000 ml of Klean-Prep^{®} intestinal lavage solution (Norgine; Marburg, Germany) were administered via the same nasojejunal tube. In order to validate the depletion of the donor's gut microbiota, samples of the primary IMEnT preparation were subjected to a standard battery of aerobic and anaerobic sterility and culture tests at the Department of Infection Medicine of a major German university hospital . No growth of any aerobic or anaerobic microbiota was observed in the primary IMEnT preparation.

### Example 4:

A fresh primary IMEnT preparation according to the present invention was produced as described in Example 3. Part of the microbiota-depleted (200 nm-filtrated) preparation was further processed by a second filtration step according to the invention by using a Mini Kleenpak^{™} Capsule Filterability Tool with Pegasus^{™} SV4 Virus Removal Filter Membrane (PALL; Dreieich, Germany) with a defined pore size of 20 nm in the air pressure filtration unit described in Example 3. The liquid primary (200 nm) and secondary (20 nm) IMEnT preparations were frozen and stored at -20°C or -80°C until lyophilisation. Freeze-drying was performed using a Sublimator 2x3x3 (Zirbus Technology; Bad Grund, Germany). The frozen liquid primary and secondary IMEnT preparations were tempered to -55°C and then subjected to the standard Sublimator freezing programme no. 1 with the following consecutive drying levels and settings:
- D01: 0.8 mbar, -25°C, 1 min (according to the setting, this stage is to be reached as quickly as possible and required approximately 60 min);
- D02: 0.5 mbar, 20°C, 720 min (12 h);
- D03: 0.1 mbar, 20°C, 360 min (6 h)
- D04: 0.05 mbar, 20°C 120 min (2 h)

The resulting lyophilised secondary IMEnT preparations were light brown, flaky powders with a significantly reduced unpleasant odor compared to conventional stool preparations. In the pilot experiments, the dry mass was approximately 0.5-1% (average: 0.7%) (w/v) with no significant difference between the 200-nm and 20-nm filtrates.

Part of the lyophilised secondary IMEnT preparations was manually encapsulated in standard size 0 gelatin capsules (content: 0.68 ml; length: 21.2 mm; WEPA, Hillscheid, Germany). Without further processing or formulation, the lyophilisate from 100 ml liquid IMEnT preparation could be encapsulated in 3-4 size 0 capsules.

### Example 5:

In this example, three typical cases of IMEnT treatment are summarised.

### Patient 1: recurrent CDI

Patient 1 was a 59-old Caucasian female who presented with three episodes of recurrent CDI over a 6-month period (confirmed by positive testing for C. *difficile* glutamate dehydrogenase and C. *difficile* toxins A and B). The patient's first episode of CDI had occurred after sigma resection due to life-threatening recurrent diverticulitis with severe bleeding which had required multiple antibiotic interventions with ciprofloxacin and metronidazole. At the initial diagnosis of CDI, the patient suffered from C. *difficile* pseudomembraneous colitis. After comprehensive screening, the patient was treated with a primary IMEnT preparation using stool donated by the patient's son according to Example 3. After IMEnT treatment, the patient felt well and could be released from the hospital on the same day. No C. *difficile* was detectable at 4 weeks post procedure. The patient had no further diarrhea, regained her normal weight and remained symptom-free at the last evaluation (more than 12 months after treatment). The example of Patient 1 shows the efficacy of IMEnT in a typical case of recurrent CDI.

### Patient 2: atypical chronic diarrhea with initial CDI and failed FMT

Patient 2 was a 73-old Caucasian female who had suffered for years from recurrent foetid diarrhea and abdominal pain with negative test results for C. *difficile* despite initial C. *difficile* detection and treatment of CDI, clinical symptoms reminiscent of recurrent CDI and potential risk factors for CDI [gastrectomy and chemotherapy (gastric carcinoma) 7 years earlier, acute diverticulitis requiring antibiotic intervention 1 year earlier]. Extensive diagnostic testing did not detect C. *difficile* or any other enteropathogen or parasite in this patient. In the hope to alleviate the chronic diarrhea by transplanting a functional gut microbiota, a conventional FMT was performed using stool donated by the husband of the patient after comprehensive screening. The FMT procedure was the same as described in Example 3, albeit without the additional filtration steps according to the present invention. Thus, the patient received a nasojejunal tube administration of pre-filtered microbiota-containing slurry after 1,000 ml of Klean-Prep^{®} intestinal lavage solution. The FMT resulted in slightly elevated body temperature (up to 38°C) for approximately 2 h after FMT and in watery diarrhea for 2 days after FMT. For approximately 2 weeks, the patient reported significant alleviation of symptoms including normalisation of stool frequency and consistency (max. 2 formed stools per day). However, the diarrhea and abdominal pain recurred after this period, and 3 months after FMT, the patient presented with essentially the same symptoms as before. At that time, IMEnT as a novel treatment option was performed as described in Example 3, again using the husband of the patient as donor. The patient felt well after IMEnT and could be released from the hospital on the same day. Despite using the same donor and recipient in the same clinical setting, IMEnT did not cause side effects such as elevated body temperature or diarrhea, indicating that these had been due to the live microbiota contained in the FMT preparation. The patient remained symptom-free at the last evaluation (7 months after treatment). The example of Patient 2 shows the efficacy of IMEnT in an atyipcal case of recurrent diarrhea with CDI-like symptoms as well as side effects and failure of conventional FMT using the same stool donor.

### Patient 3: recurrent CDI with multimorbidity and immunosuppression

Patient 3 was a 72-old Caucasian male who was hospitalised in early 2014 because of acute C. difficile-associated diarrhea, which was his second relapse of CDI. Three years earlier, CDI had led to sepsis in this patient. The patient was multimorbid and under ciclosporine immunosuppression after a kidney transplant in 1990 due to cirrhosis of both kidneys. In addition, the patient suffered from severe coronary heart disease with multiple stent implants and had a high-risk profile for cardiovascular disease (arterial hypertension, dyslipoproteinemia and nicotine abuse). The patient had experienced numerous infectious complications under immunosuppressive therapy, partly with pre-terminal renal failure. The current episode of CDI was accompanied by a significant elevations of renal retention parameters (pre-terminal renal failure) and required hospitalisation. The CDI was treated with a dual therapy of vancomycin and rifaximin together with Perenterol^{®} (dry yeast capsules, *Saccharomyces boulardii*; MEDICE; Iserlohn, Germany), but the diarrhea persisted. CDI was confirmed by positive testing for C. *difficile* glutamate dehydrogenase and toxin-producing C. *difficile.* For treating this patient, IMEnT rather than conventional FMT was chosen due to the increased risk of intestinal bacterial translocation under immunosuppression. After comprehensive screening, the patient was treated with a primary IMEnT preparation according to Example 3 using stool donated by the patient's sister. After IMEnT treatment, the patient felt well and remained symptom-free at the last evaluation (7 months after treatment). The example of Patient 3 shows the efficacy of IMEnT in a case of recurrent CDI complicated by multimorbidity and immunosuppression.

### Example 6:

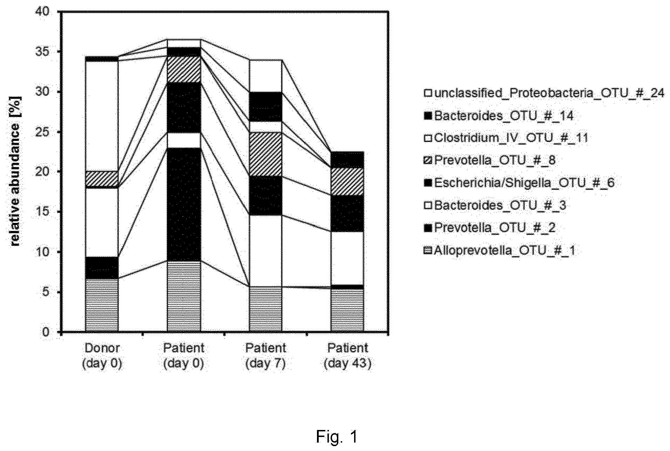

Due to the particularly interesting case of Patient 2 (see Example 5), stool samples of this patient and the donor (her husband) were subjected to a microbiome analysis. As shown in Figure 1, the IMEnT treatment led to a profound and lasting change in the microbiome of the patient.

The following methodology was used for microbiome analysis:
Genomic DNA from fecal samples was extracted using the PowerSoil^{®} DNA Isolation Kit (MO BIO, Carlsbad, CA, USA) according to the manufacturer's instructions (Rehman et al. 2011 Gut 60:1354) with some modifications: solution C1 and 20 µL of Proteinase K were added to the feces and incubated for 2 h at 50°C to enhance the lysis. Extracted DNA was quantified using the Quant-iT^{™} PicoGreen dsDNA Assay Kit (Life Technologies/Invitrogen, Darmstadt, Germany).

The 16S rRNA gene variable region V4 was PCR-amplified using dual indexed primers (515F and 806R). Forward primers included the 5' Illumina adapter <AATGATACGGCGACCACCGAGATCTACAC>, forward primer pad <TATGGTAATT>, linker <GT> and 16S rRNA gene-specific primer <GTGCCAGCMGCCGCGGTAA>, and the reverse primer consisted of the reverse complement of the 3' Illumina adapter <CAAGCAGAAGACGGCATACGAGAT>, primer pad < AGTCAGTCAG>, linker <CC> and 16S rRNA gene-specific reverse primer <GGACTACHVGGGTWTCTAAT> (Caporaso et al. 2012 ISME J. 6:1621). 2 µl of DNA was amplified using the composite primers in duplicate in a GeneAmp PCR system 9700 (Life Technologies/Applied Biosystems, Darmstadt, Germany) using the following cycling conditions: an initial denaturation of 3 min at 98°C followed by 30 cycles with denaturation at 98°C for 10 s, annealing at 50°C for 30 s and elongation at 72°C for 30 s. Final extension was performed at 72°C for 10 min. The amplified product was run on an agarose gel to assess the amplicon size and amplification performance. Amplicons quantities were normalized using the SequalPrep^{™} kit (Life Technologies/Invitrogen). Specific bands were excised and purified using the QIAquick gel extraction kit (Qiagen, Hilden, Germany). The concentration of purified amplicons was measured with the Quant-iT^{™} PicoGreen dsDNA Assay Kit. Water was used as a negative control for PCR and did not result in quantifiable templates. Equal amounts of PCR products were mixed in a single tube and sequenced on a Illumina MiSeq using a 2 x 300 sequencing kit (Illumina, San Diego, CA, USA), which allowed to generate paired end reads with overlapping regions.

Sequencing reads were primarily processed for quality control using the software Mothur (Schloss et al. 2009 Appl. Environ. Microbiol. 75:7537). Forward and reverse reads were assembled to form contigs. Sequences with more than 450 bases, having any ambiguous base or more than 6 homopolymers were removed from downstream analysis. Chimeric sequences were detected with the Uchime algorithm and removed. Sequences were classified using the Mothur formated training sets of the Ribosomal Database Project reference and taxonomy. Sequences classified as eukaryotes, chloroplast and mitochondria were removed from analysis. Remaining good quality sequences were aligned and a distance matrix was computed. Sequences with at least 97% similarity were clustered in into operational taxonomical units (OTUs) using Mothur. These OTUs were classified taxonomically using the Mothur modified RDP reference and taxonomy database and RDP-based SeqMatch (for species level identification only). Principle coordinate analysis was performed on abundance and non-abundance-based distance matrices generated in the PAST software (Hammer et al. 2001 Palaeontol. Electron. 4:1 :9pp).

### Example 7:

In this example, the suitability of stool from specific pathogen-free Göttingen minipigs (Ellegaard Göttingen Minipigs A/S, Dalmose, Denmark) for IMEnT preparations was investigated. Such pigs are bred and kept under good manufacturing practice (GMP) conditions with defined food, free of pathogens and with only minimal factors of variance, which offers enormous advantages over using a human donor pool. In particular, the scalability, the controlled food regimen (without allergens, but with an optimal composition for the intestinal microbiota and, if desired, controlled supplementation of food ingredients, prebiotics, probiotics and the like) as well as the absence of human risk factors enables the production of large, homogeneous and completely characterisable stool (and IMEnT) batches even under pharmaceutical GMP production conditions. Stool from pigs is harvested in regular intervals several times a day and either processed immediately or stored in an airtight container at 4°C for up to 6 hours. The principle procedure for producing the primary IMEnT preparation from pig stool is the same as described in Example 3 for human stool, albeit the amounts and production machinery are upscaled significantly, depending on the required batch size.

For the present example in an experimental setting, the same procedure as described in Example 3 was used to prepare and analyse stool samples from 9 minipigs (5 male, 4 female). The objective of the pilot study was to characterize the microbiota of the minipigs in terms of inter-individual variability and microbial diversity and to confirm the published similarity to the human intestinal microbiota (Leser et al. 2002 Appl. Environ. Microbiol. 68:673; Lamendella et al. 2011 BMC Microbiol. 11:103). The methodology for microbiota analysis was the same as described in Example 6.

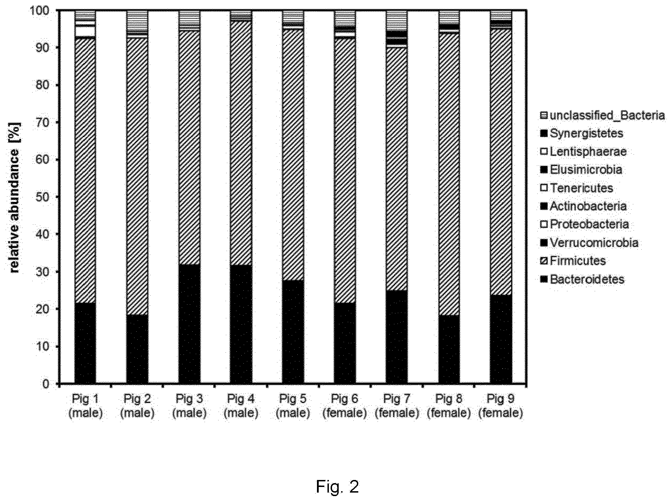

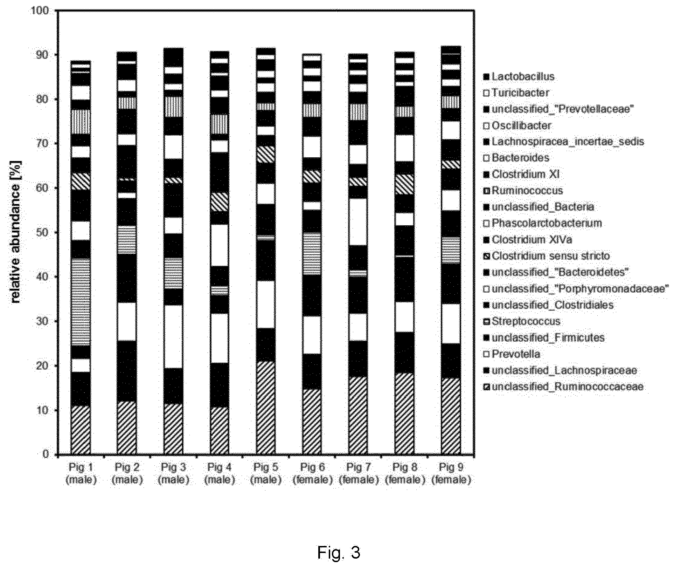

The results of the analyses (Figures 2 and 3) show that the stool microbiota of specific pathogen-free Göttingen minipigs bred and kept under GMP conditions has a very low inter-individual variability and sufficient diversity on the phylum level and even on the genus level. Moreover, the stool microbiota of these pigs strongly resembles human stool microbiota (as published in the literature), which supports the use of stool of such pigs for IMEnT preparations according to the present invention.

## Claims

1. A preparation from animal and/or artificial and/or synthetic and/or cultured and/or fermented stool for use as a medicine, in form of a filtrate made by suspending the stool in a liquid and by depletion of the microbiota comprised in the suspension by filtrating the suspension with a filter having a maximum pore size of ≤ 450 nm to obtain a filtrate of an intestinal microenvironment that is a microbiota-depleted liquid phase of the stool preparation, and which contains a mixture of metabolites, nutrients and mediators, produced by both the intestinal microbiota and the donor' body cells, to positively influence and/or revitalise and/or normalise the residual intestinal microbiota in a recipient.

2. A preparation from animal and/or artificial and/or synthetic and/or cultured and/or fermented stool for use as a medicine according to claim 1, wherein in the preparation in form of a filtrate the water content is ≤ 95 weight-% relating to the total weight of the filtrate and/or the microbiota and the virus load comprised in the suspension is depleted by performing a second filtration with a maximum pore size of ≤ 20 nm.

3. The preparation for use as a medicine according to claim 1 or 2, wherein the animal is a mammal, preferably a pig or human.

4. The preparation for use as a medicine according to any of claims 1-3, wherein the stool is from a pooled sample.

5. A preparation from animal, preferably mammal, in particular pig or human, stool and/or artificial and/or synthetic and/or cultured and/or fermented stool, in the form of a dietary supplement, in form of a filtrate made by suspending the stool in a liquid and by depletion of the microbiota comprised in the suspension by filtrating the suspension with a filter having a maximum pore size of ≤ 450 nm to obtain a filtrate of an intestinal microenvironment that is a microbiota-depleted liquid phase of the stool preparation, and which contains a mixture of metabolites, nutrients and mediators, produced by both the intestinal microbiota and the donor' body cells, to positively influence and/or revitalise and/or normalise the residual intestinal microbiota in a recipient who is a healthy consumer in terms of not having a specific disease.

6. The preparation from animal and/or artificial and/or synthetic and/or cultured and/or fermented stool in the form of a dietary supplement according to claim 5, wherein in the preparation in form of a filtrate the water content is ≤ 95 weight-% relating to the total weight of the filtrate and/or the microbiota and the virus load comprised in the suspension is depleted by performing a second filtration with a maximum pore size of ≤ 20 nm.

7. The preparation in the form of a dietary supplement according to claim 5 or 6, wherein the animal is a mammal, preferably a pig or human.

8. The preparation in the form of a dietary supplement according to any of claims 5-7, wherein the stool is from a pooled sample.

9. The preparation as defined in any of claims 1-4 for use in the therapy or prophylaxis of diseases that result from changes in the intestinal microbiota and/or an impaired interaction between intestinal microbiota and intestines.

10. The preparation for use according to claim 9, wherein the disease is selected from the group consisting of autoimmune, intestinal, metabolic, cardiovascular, atopic, allergic, neurodevelopmental, neurodegenerative, idiopathic and chronic pain diseases, preferably selected from the group consisting of intestinal inflammatory and/or infectious diseases, especially *Clostridium difficile* infection (CDI); pseudomembraneous colitis resulting from *Clostridium difficile* toxins; other chronic gastrointestinal infections *e.g.,* by *Yersinia* spp., *Campylobacter* spp., *Aeromonas* spp., *Escherichia coli, Cryptosporidium* spp., *Shigella* spp., amoebae, Giardia, or chronic viral infections; small bowel bacterial overgrowth, inflammatory bowel diseases (IBD), Crohn's disease, ulcerative colitis, lymphocytic colitis, mucous colitis, collageneous colitis, microscopic colitis, antibiotic-associated colitis, enterohemorrhagic colitis, diverticular disease, AIDS enteropathy, irritable bowel syndrome, spastic colon, chronic diarrhea, idiopathic or simple or chronic constipation, eosinophilic disorders of the gastrointestinal tract, sacroiliitis, metabolic syndrome, obesity, diabetes, insulin resistance, non-alcoholic fatty liver disease, non-alcoholic steatohepatitis, cardiovascular diseases, arteriosclerosis, atherosclerosis, coronary heart disease, autoimmune diseases, atopic diseases, allergic diseases, atopic eczema, asthma, psoriasis, cystic fibrosis, COPD, systemic autoimmunity including rheumatoid arthritis and systemic lupus erythematosus, idiopathic thrombocytopenic purpura, chronic pain disorders such as fibromyalgia, chronic fatigue syndrome, eating disorders, malnutrition, and neurodevelopmental or neurodegenerative disorders, such as Parkinson's disease, multiple sclerosis or autism.

11. The preparation for use as a medicine according to any of claims 1-4 formulated as a pharmaceutical composition for oral administration, preferably with controlled and/or delayed release, of the active ingredients for specific local effectiveness in the small intestine and/or colon.

12. The preparation for use as a medicine according to any of claims 1-4 formulated as a pharmaceutical composition for (neo)rectal administration in the colon or pouch.

13. The preparation for use as a medicine according to any of claims 1-4 formulated as a pharmaceutical composition for nasal administration.

14. The preparation for use formulated as a pharmaceutical composition according to any of claims 11-13 comprising one or more substances selected from the group consisting of probiotics; prebiotics; synbiotics; proteins and/or enzymes supporting probiotics; antibiotic, antimycotic, antiprotozoal, antihelminthic, antiviral or anti-inflammatory agents; aminosalicylates; nicotinic acid; nicotinamide; tryptophan; compounds that convert in the body of an animal or human into nicotinic acid, nicotinamide or tryptophan; nicotinamide adenine dinucleotide (NAD); nicotinamide adenine dinucleotide phosphate (NADP); intermediates in the biosynthesis of NAD or NADP; tryptophan dipeptides; short-chain fatty acids; medium-chain fatty acids; systemic or topical corticosteroids; β₂-adrenergic receptor agonists; theophylline and other substances of the xanthine family; statins; small molecules; peptides and biologicals.

15. A method for preparing a preparation in form of a filtrate as defined in any of claims 1-8, comprising the steps of:
a) providing an animal, preferably a mammal, in particular a pig or human, stool sample,
b) suspending the stool sample,
c) depleting the microbiota comprised in the suspension by filtrating the suspension with a filter having a maximum pore size of ≤ 450 nm resulting in a filtrate of an intestinal microenvironment to obtain a filtrate that is a microbiota-depleted liquid phase of the stool preparation, and which contains a mixture of metabolites, nutrients and mediators, produced by both the intestinal microbiota and the donor' body cells, to positively influence and/or revitalise and/or normalise the residual intestinal microbiota in a recipient; and preferably
d) reducing the water content of the preparation to ≤ 95 weight-% relating to the total weight of the preparation and/or depleting the microbiota comprised in the suspension by performing a second filtration with a maximum pore size of ≤ 20 nm.

## Patentansprüche

1. Präparat aus tierischem und/oder künstlichem und/oder synthetischem und/oder kultiviertem und/oder fermentiertem Stuhl zur Anwendung als Arzneimittel, in Form eines Filtrats, das durch Suspendieren des Stuhls in einer Flüssigkeit und durch Abreicherung der in der Suspension enthaltenen Mikrobiota durch Filtrieren der Suspension mit einem Filter mit einer maximalen Porengröße von ≤ 450 nm hergestellt wurde, um ein Filtrat einer Darmmikroumgebung zu erhalten, das eine an Mikrobiota abgereicherte flüssige Phase des Stuhlpräparats ist, und das eine Mischung aus Metaboliten, Nährstoffen und Überträgerstoffen enthält, die sowohl von der Darmmikrobiota als auch von den Körperzellen des Spenders produziert wurden, um die restliche Darmmikrobiota in einem Empfänger positiv zu beeinflussen und/oder zu revitalisieren und/oder zu normalisieren.

2. Präparat aus tierischem und/oder künstlichem und/oder synthetischem und/oder kultiviertem und/oder fermentiertem Stuhl zur Anwendung als Arzneimittel nach Anspruch 1, wobei der Wassergehalt in dem Präparat in Form eines Filtrats ≤ 95 Gew.-%, bezogen auf das Gesamtgewicht des Filtrats und/oder der Mikrobiota, beträgt und die in der Suspension enthaltene Viruslast durch Durchführen einer zweiten Filtration mit einer maximalen Porengröße von ≤ 20 nm abgereichert ist.

3. Präparat zur Anwendung als Arzneimittel nach Anspruch 1 oder 2, wobei das Tier ein Säugetier, vorzugsweise ein Schwein oder ein Mensch, ist.

4. Präparat zur Anwendung als Arzneimittel nach einem der Ansprüche 1 - 3, wobei der Stuhl aus einer Sammelprobe stammt.

5. Präparat aus tierischem, vorzugsweise Säugetier-, insbesondere Schweine- oder menschlichem, Stuhl und/oder künstlichem und/oder synthetischem und/oder kultiviertem und/oder fermentiertem Stuhl, in Form eines Nahrungsergänzungsmittels, in Form eines Filtrats, das durch Suspendieren des Stuhls in einer Flüssigkeit und durch Abreicherung der in der Suspension enthaltenen Mikrobiota durch Filtrieren der Suspension mit einem Filter mit einer maximalen Porengröße von ≤ 450 nm hergestellt wurde, um ein Filtrat einer Darmmikroumgebung zu erhalten, das eine an Mikrobiota abgereicherte flüssige Phase des Stuhlpräparats ist, und das eine Mischung aus Metaboliten, Nährstoffen und Überträgerstoffen enthält, die sowohl von der Darmmikrobiota als auch von den Körperzellen des Spenders produziert wurden, um die restliche Darmmikrobiota eines Empfängers, der ein gesunder Konsument ist, d.h. keine spezifische Krankheit hat, positiv zu beeinflussen und/oder zu revitalisieren und/oder zu normalisieren.

6. Präparat aus tierischem und/oder künstlichem und/oder synthetischem und/oder kultiviertem und/oder fermentiertem Stuhl in Form eines Nahrungsergänzungsmittels nach Anspruch 5, wobei in dem Präparat in Form eines Filtrats der Wassergehalt ≤ 95 Gew.-%, bezogen auf das Gesamtgewicht des Filtrats und/oder der Mikrobiota, beträgt und die in der Suspension enthaltene Viruslast durch Durchführen einer zweiten Filtration mit einer maximalen Porengröße von ≤ 20 nm abgereichert ist.

7. Zubereitung in Form eines Nahrungsergänzungsmittels nach Anspruch 5 oder 6, wobei das Tier ein Säugetier, vorzugsweise ein Schwein oder ein Mensch ist.

8. Präparat in Form eines Nahrungsergänzungsmittels nach einem der Ansprüche 5-7, wobei der Stuhl aus einer Sammelprobe stammt.

9. Präparat wie in einem der Ansprüche 1-4 definiert, zur Anwendung in der Therapie oder Prophylaxe von Krankheiten, die aus Veränderungen der Darmmikrobiota und/oder einer gestörten Interaktion zwischen Darmmikrobiota und Darm resultieren.

10. Präparat zur Anwendung nach Anspruch 9, wobei die Krankheit aus der Gruppe ausgewählt ist, die aus Autoimmun-, Darm-, Stoffwechsel-, Herz-Kreislauf-, atopischen, allergischen, neuroentwicklungsbedingten, neurodegenerativen, idiopathischen und chronischen Schmerzkrankheiten besteht, vorzugsweise ausgewählt aus der Gruppe, die aus entzündlichen und/oder infektiösen Darmerkrankungen, insbesondere *Clostridium difficile* Infektion (CDI); pseudomembranöser Kolitis, die aus *Clostridium difficile* Toxinen resultiert; anderen chronischen gastrointestinalen Infektionen, *z.B*., durch *Yersinia* spp., *Campylobacter* spp., *Aeromonas* spp., *Escherichia coli, Cryptosporidium* spp., *Shigella* spp, Amöben, Giardia, oder chronischen Virusinfektionen; bakterieller Überbesiedelung des Dünndarms, entzündlichen Darmerkrankungen (IBD), Morbus Crohn, Colitis ulcerosa, lymphozytärer Colitis, schleimiger Colitis, kollagener Colitis, mikroskopischer Colitis, Antibiotika-assoziierter Colitis, enterohämorrhagischer Colitis, Divertikelkrankheit, AIDS-Enteropathie, Reizdarmsyndrom, spastischem Dickdarm, chronischem Durchfall, idiopathischer oder einfacher oder chronischer Verstopfung, eosinophilen Erkrankungen des Magen-Darm-Trakts, Sakroiliitis, metabolischem Syndrom, Fettleibigkeit, Diabetes, Insulinresistenz, nichtalkoholischer Fettlebererkrankung, nichtalkoholischer Steatohepatitis, Herz-Kreislauf-Erkrankungen, Arteriosklerose, Atherosklerose, koronarer Herzkrankheit, Autoimmunerkrankungen, atopischen Erkrankungen, allergischen Erkrankungen, atopischem Ekzem, Asthma, Psoriasis, zystischer Fibrose, COPD, systemischer Autoimmunität einschließlich rheumatoider Arthritis und systemischem Lupus erythematodes, idiopathischer Thrombozytopenie, chronischen Schmerzstörungen wie Fibromyalgie, chronischem Müdigkeitssyndrom, Essstörungen, Unterernährung und neuroentwicklungsbedingten oder neurodegenerativen Störungen wie Parkinson, Multiple Sklerose oder Autismus besteht.

11. Präparat zur Anwendung als Arzneimittel nach einem der Ansprüche 1-4, formuliert als eine pharmazeutische Zusammensetzung zur oralen Verabreichung, vorzugsweise mit kontrollierter und/oder verzögerter Freisetzung, der Wirkstoffe für eine spezifische lokale Wirksamkeit im Dünndarm und/oder Dickdarm.

12. Präparat zur Anwendung als Arzneimittel nach einem der Ansprüche 1-4, formuliert als eine pharmazeutische Zusammensetzung zur (neo)rektalen Verabreichung in den Dickdarm oder eine Gewebetasche.

13. Präparat zur Anwendung als Arzneimittel nach einem der Ansprüche 1-4, formuliert als eine pharmazeutische Zusammensetzung zur nasalen Verabreichung.

14. Zubereitung zur Anwendung, formuliert als pharmazeutische Zusammensetzung nach einem der Ansprüche 11-13, umfassend eine oder mehrere Substanzen, ausgewählt aus der Gruppe, bestehend aus Probiotika; Präbiotika; Synbiotika; Proteinen und/oder Enzymen, die Probiotika unterstützen; antibiotischen, antimykotischen, antiprotozoischen, antihelminthischen, antiviralen oder entzündungshemmenden Mitteln; Aminosalicylaten; Nikotinsäure; Nikotinamid; Tryptophan; Verbindungen, die sich im Körper eines Tieres oder eines Menschen in Nikotinsäure, Nikotinamid oder Tryptophan umwandeln; Nikotinamid-Adenin-Dinukleotid (NAD); Nikotinamid-Adenin-Dinukleotid-Phosphat (NADP); Zwischenprodukten in der Biosynthese von NAD oder NADP; Tryptophan-Dipeptiden; kurzkettigen Fettsäuren; mittelkettigen Fettsäuren; systemischen oder topischen Kortikosteroiden; β₂-adrenergen Rezeptor-Agonisten; Theophyllin und anderen Substanzen der Xanthin-Familie; Statinen; kleinen Molekülen; Peptiden und Biologicals.

15. Verfahren zum Herstellen eines Präparats in Form eines Filtrats, wie in einem der Ansprüche 1-8 definiert, umfassend die Schritte:
a) Bereitstellen einer Stuhlprobe eines Tieres, vorzugsweise eines Säugetiers, insbesondere eines Schweins oder eines Menschen,
b) Suspendieren der Stuhlprobe,
c) Abreichern der in der Suspension enthaltenen Mikrobiota durch Filtrieren der Suspension mit einem Filter mit einer maximalen Porengröße von ≤ 450 nm, was zu einem Filtrat einer Darmmikroumgebung führt, um ein Filtrat zu erhalten, das eine an Mikrobiota abgereicherte flüssige Phase des Stuhlpräparats ist und das eine Mischung von Metaboliten, Nährstoffen und Überträgerstoffen enthält, die sowohl von der Darmmikrobiota als auch von den Körperzellen des Spenders produziert wurden, um die restliche Darmmikrobiota in einem Empfänger positiv zu beeinflussen und/oder zu revitalisieren und/oder zu normalisieren; und vorzugsweise
d) Reduzieren des Wassergehalts des Präparats auf ≤ 95 Gew.-%, bezogen auf das Gesamtgewicht des Präparats, und/oder Abreichern der in der Suspension enthaltenen Mikrobiota durch Durchführen einer zweiten Filtration mit einer maximalen Porengröße von ≤ 20 nm.

## Revendications

1. Préparation à partir de selles animales et/ou artificielles et/ou synthétiques et/ou de culture et/ou fermentées pour une utilisation comme médicament, sous forme d'un filtrat fabriqué par suspension des selles dans un liquide et par appauvrissement du microbiote compris dans la suspension par filtration de la suspension avec un filtre présentant une taille de pores maximale ≤ 450 nm pour obtenir un filtrat d'un microenvironnement intestinal qui est une phase liquide appauvrie en microbiote de la préparation de selles, et qui contient un mélange de métabolites, nutriments et médiateurs, produits par à la fois le microbiote intestinal et les cellules corporelles du donneur, pour influencer positivement et/ou revitaliser et/ou normaliser le microbiote intestinal résiduel chez un receveur.

2. Préparation à partir de selles animales et/ou artificielles et/ou synthétiques et/ou de culture et/ou fermentées pour une utilisation comme médicament selon la revendication 1, dans laquelle dans la préparation sous forme d'un filtrat la teneur en eau est ≤ 95 % en poids par rapport au poids total du filtrat et/ou du microbiote et la charge virale comprise dans la suspension est appauvrie par réalisation d'une deuxième filtration avec une taille de pores maximale ≤ 20 nm.

3. Préparation pour une utilisation comme médicament selon la revendication 1 ou 2, dans laquelle l'animal est un mammifère, de préférence un cochon ou humain.

4. Préparation pour une utilisation comme médicament selon l'une quelconque des revendications 1-3, dans laquelle les selles proviennent d'un échantillon groupé.

5. Préparation à partir de selles animales, de préférence de mammifère, en particulier de cochon ou d'humain et/ou de selles artificielles et/ou synthétiques et/ou de culture et/ou fermentées sous forme d'un complément alimentaire, sous forme d'un filtrat fabriqué par suspension des selles dans un liquide et par appauvrissement du microbiote compris dans la suspension par filtration de la suspension avec un filtre présentant une taille de pores maximale ≤ 450 nm pour obtenir un filtrat d'un microenvironnement intestinal qui est une phase liquide appauvrie en microbiote de la préparation de selles, et qui contient un mélange de métabolites, nutriments et médiateurs, produits par à la fois le microbiote intestinal et les cellules corporelles du donneur, pour influencer positivement et/ou revitaliser et/ou normaliser le microbiote intestinal résiduel chez un receveur qui est un consommateur en bonne santé en termes d'absence de maladie spécifique.

6. Préparation à partir de selles animales et/ou artificielles et/ou synthétiques et/ou de culture et/ou fermentées sous forme d'un complément alimentaire selon la revendication 5, dans laquelle dans la préparation sous forme d'un filtrat la teneur en eau est ≤ 95 % en poids par rapport au poids total du filtrat et/ou du microbiote et la charge virale comprise dans la suspension est appauvrie par réalisation d'une deuxième filtration avec une taille de pores maximale ≤ 20 nm.

7. Préparation sous la forme d'un complément alimentaire selon la revendication 5 ou 6, dans laquelle l'animal est un mammifère, de préférence un cochon ou humain.

8. Préparation sous la forme d'un complément alimentaire selon l'une quelconque des revendications 5-7, dans laquelle les selles proviennent d'un échantillon groupé.

9. Préparation selon l'une quelconque des revendications 1-4 pour une utilisation dans la thérapie ou la prophylaxie de maladies qui proviennent de changements dans le microbiote intestinal et/ou d'une interaction détériorée entre le microbiote intestinal et les intestins.

10. Préparation pour une utilisation selon la revendication 9, dans laquelle la maladie est choisie dans le groupe constitué des maladies auto-immunes, intestinales, métaboliques, cardiovasculaires, atopies, allergiques, neurodéveloppementales, neurodégénératives, idiopathiques et à douleur chronique, de préférence choisie dans le groupe constitué des maladies inflammatoires intestinales et/ou infectieuses, en particulier l'infection à Clostridium difficile (CDI) ; la colite pseudomembraneuse résultant des toxines Clostridium difficile ; d'autres infections gastrointestinales chroniques par exemple, par Yersinia spp., Campylobacter spp., Aeromonas spp., Escherichia coli, Cryptosporidium spp., Shigella spp., amibes, Giardia, ou des infections virales chroniques ; pullulation bactérienne dans l'intestin grêle, maladies inflammatoires de l'intestin (IBD), maladie de Crohn, rectocolite hémorragique, colite lymphocytaire, colite muqueuse, colite collagéneuse, colite microscopique, colite associée à un antibiotique, colite entéro-hémorragique, maladie diverticulaire, entéropathie associée au SIDA, syndrome de l'intestin irritable, côlon irritable, diarrhée chronique, constipation idiopathique ou simple ou chronique, troubles éosinophiles du tractus digestif, sacro-illite, syndrome métabolique, obésité, diabète, résistance à l'insuline, maladie du foie gras non alcoolique, stéatohépatite non alcoolique, maladies cardiovasculaires, artériosclérose, athérosclérose, maladie coronarienne, maladies auto-immunes, atopies, maladies allergiques, eczéma atopique, asthme, psoriasis, fibrose cystique, BPCO, auto-immunité systémique comportant la polyarthrite rhumatoïde et le lupus érythémateux systémique, purpura thrombocytopénique idiopathique, troubles à douleur chronique tels que la fibromyalgie, syndrome de fatigue chronique, troubles du comportement alimentaire, malnutrition, et troubles du développement neurologique ou neurodégénératifs, tels que la maladie de Parkinson, la sclérose en plaques ou l'autisme.

11. Préparation pour une utilisation comme médicament selon l'une quelconque des revendications 1-4 formulée comme une composition pharmaceutique pour une administration orale, de préférence à libération contrôlée et/ou retardée, des ingrédients actifs pour une efficacité locale spécifique dans l'intestin grêle et/ou le colon.

12. Préparation pour une utilisation comme médicament selon l'une quelconque des revendications 1-4 formulée comme une composition pharmaceutique pour une administration (néo)rectale dans le colon ou une poche.

13. Préparation pour une utilisation comme médicament selon l'une quelconque des revendications 1-4 formulée comme une composition pharmaceutique pour une administration nasale.

14. Préparation pour une utilisation formulée comme une composition pharmaceutique selon l'une quelconque des revendications 11-13 comprenant une ou plusieurs substances choisies dans le groupe constitué des probiotiques ; prébiotiques ; synbiotiques ; probiotiques supportant des protéines et/ou enzymes ; agents antibiotiques, antimycotiques, antiprotozoaires, antihelminthiques, antiviraux ou anti-inflammatoires ; aminosalicylates ; acide nicotinique ; nicotinamide ; tryptophane ; composés qui se convertissent dans le corps d'un animal ou humain en acide nicotinique, nicotinamide ou tryptophane ; nicotinamide adénine dinucléotide (NAD) ; nicotinamide adénine dinucléotide phosphate (NADP) ; intermédiaires dans la biosynthèse de NAD ou NADP ; dipeptides de tryptophane ; acides gras à chaîne courte ; acides gras à chaîne moyenne ; corticostéroïdes systémiques ou topiques ; agonistes du récepteur β₂-adrénergique; théophylline et d'autres substances de la famille xanthine ; statines ; petites molécules ; peptides et produits biologiques.

15. Procédé pour préparer une préparation sous forme d'un filtrat telle que définie dans l'une quelconque des revendications 1-8, comprenant les étapes de :
a) fourniture d'un échantillon de selles d'animal, de préférence de mammifère, en particulier de cochon ou d'humain,
b) suspension de l'échantillon de selles,
c) appauvrissement du microbiote compris dans la suspension par filtration de la suspension avec un filtre présentant une taille de pores maximale ≤ 450 nm entraînant un filtrat d'un microenvironnement intestinal pour obtenir un filtrat qui est une phase liquide appauvrie en microbiote de la préparation de selles, et qui contient un mélange de métabolites, nutriments et médiateurs, produit par à la fois le microbiote intestinal et les cellules corporelles du donneur, pour influencer positivement et/ou revitaliser et/ou normaliser le microbiote intestinal résiduel chez un receveur ; et de préférence
d) réduction de la teneur en eau de la préparation jusqu'à ≤ 95 % en poids par rapport au poids total de la préparation et/ou appauvrissement du microbiote compris dans la suspension par réalisation d'une seconde filtration avec une taille de pores maximale ≤ 20 nm.


### SEQUENCE LISTING

<110> CONARIS RESEARCH INSTITUTE AG
<120> Methods and compositions for intestinal microenvironment transfer
<130> CA 1764-03WO
<150> EP14179541.9
   <151> 2014-08-01
   <150> EP14155648.0-1456
   <151> 2014-02-18
<160> 6
<170> BiSSAP 1.3
<210> 1
   <211> 29
   <212> DNA
   <213> Artificial Sequence
<220>
   <223> 1
<400> 1
   aatgatacgg cgaccaccga gatctacac 29
<210> 2
   <211> 10
   <212> DNA
   <213> Artificial Sequence
<220>
   <223> 2
<400> 2
   tatggtaatt 10
<210> 3
   <211> 19
   <212> DNA
   <213> Artificial Sequence
<220>
   <223> 3
<400> 3
   gtgccagcmg ccgcggtaa 19
<210> 4
   <211> 24
   <212> DNA
   <213> Artificial Sequence
<220>
   <223> 4
<400> 4
   caagcagaag acggcatacg agat 24
<210> 5
   <211> 10
   <212> DNA
   <213> Artificial Sequence
<220>
   <223> 5
<400> 5
   agtcagtcag 10
<210> 6
   <211> 20
   <212> DNA
   <213> Artificial Sequence
<220>
   <223> 6
<400> 6
   ggactachvg ggtwtctaat 20